# EUROPEAN PATENT APPLICATION

(11) **EP 3 858 865 A1**
(43) Date of publication of application: **04.08.2021**
(21) Application number: 19865552.4
(22) Date of filing: 24.09.2019
(51) Int. Cl.: C07K 19/00, C12N 9/00, C12N 15/00, A61P 7/00, A61K 38/00

(54) **FUSION PROTEIN OF MUTATED SINGLE-CHAIN HUMAN COAGULATION FACTOR VIII, PREPARATION METHOD THEREFOR, AND USE THEREOF**

(30) Priority: 26.09.2018 CN 201811123918
(71) Applicant: Ampsource Biopharma Shanghai Inc., Shanghai 201318 (CN); Pharmab, Inc., Shanghai 201203 (CN)
(72) Inventor: GAO, Yongjuan, Shanghai 201203 (CN); JIA, Shixiang, Shanghai 201318 (CN); ZHENG, Yuncheng, Shanghai 201318 (CN); JIN, Yingying, Shanghai 201318 (CN); WANG, Zhu, Shanghai 201318 (CN); DONG, Zhao, Shanghai 201318 (CN); CHEN, Si, Shanghai 201318 (CN); SUN, Bill, Nai-chau, Shanghai 201318 (CN); LI, Qiang, Shanghai 201318 (CN)
(74) Representative: Prinz & Partner mbB
(86) International application number: PCT/CN2019/107432
(87) International publication number: WO 2020/063562

(57) **Abstract**

Disclosed is a fusion protein of a mutated recombinant single-chain human coagulation factor VIII (FVIII), a preparation method therefor, and a use thereof. The fusion protein sequentially comprises, from an N-terminus to a C-terminus, a mutated single-chain human FVIII having a partially deleted B-domain, a flexible peptide linker, at least one rigid unit of a carboxyl-terminal peptide of a human chorionic gonadotropin beta subunit, and a half-life prolonging moiety (preferably an IgG Fc variant). The fusion protein has a similar biological activity to a recombinant FVIII, a prolonged active half life in vivo, and better stability in vitro and in vivo, and thus improves the pharmacokinetics and efficacy of the fusion protein.

## Description

### TECHNICAL FIELD

The present disclosure relates to the field of fusion proteins and, in particular, to a fusion protein of a mutant human coagulation factor VIII (FVIII), a preparation method thereof, and use thereof, especially use for treating a variety of coagulation-related diseases.

### BACKGROUND

Coagulation factor VIII (FVIII), also referred to as an anti-hemophilic factor, plays an important role in an endogenous coagulation system. The results of numerous studies on FVIII in molecular genetics show that deficiency of FVIII in sex chromosome X-linked genes results in hemophilia A. According to statistics, the prevalence of hemophilia A in males is 1/5000 and accounts for more than 80% of the total number of hemophilia patients. At present, a common treatment for hemophilia A is replacement therapy, that is, the supplement of FVIII that hemophilia patients lack.

Mature FVIII consists of light and heavy chains with a molecular weight of approximately 280 kDa and has domains A1-A2-B-A3-C1-C2. In cells, the proteolysis of residue Arg-1648 in the B domain produces a heavy chain (A1-A2-B) with different sizes ranging from 90 to 200 kDa and a light chain (domains A3-C1-C2) of 80 kDa. The heavy chain and the light chain bind to form a heterodimer via divalent metal ion-dependent links. In plasma, the dimer consisting of a heavy chain and a light chain then binds to von Willebrand Factor (vWF) with high affinity to be protected from being degraded prior to maturation. The half-life of non-activated FVIII that binds to vWF is approximately 12 hours in plasma. FVIII is activated through hydrolytic cleavage of Arg372 and Arg740 in the heavy chain and Arg1689 in the light chain by activated coagulation factor FX (FXa) and thrombin FII (FIIa), so that vWF factor is released and activated FVIII dimers (FVIIIa) are generated. The FVIIIa forms a tight complex with activated coagulation factor FIX (FIXa) and FX on the surface of phospholipid in the presence of Ca²⁺. FX is then activated by FIXa. The activated FX is dissociated from the complex and converts prothrombin to thrombin, where thrombin can directly convert fibrinogen to fibrin. As a cofactor in the coagulation system, FVIII can increase the catalytic efficiency of FIXa for activating FX by several orders of magnitude.

Due to a complex structure and a large molecular weight of FVIII protein, FVIII is very unstable and easy to deactivate in the processes such as plasma collection and recombinant purification and preparation. FVIII takes effect within a relatively short period, especially in an aqueous solution. Factors such as storage temperature, inorganic salt ions, trace proteases, and other proteins involved in coagulation (especially vWF and albumin) all affect the stability of FVIII molecules. During injection administration after the reconstitution of FVIII lyophilisates, safety and sterility must be guaranteed, and the infusion must be completed within specified time which is generally no more than 4 hours. On the other hand, violent oscillations when the FVIII lyophilisates are reconstituted will also result in damages to the structure of proteins, which deactivates FVIII. Therefore, the development of FVIII molecules with better stability can improve the flexibility of FVIII products in clinical applications and is essential to greatly improve the medication safety and quality of life of patients.

The B domain of FVIII contains 18 N-glycosylation sites, has no known function related to coagulation, and is not homologous to other proteins. FVIII molecules with the B domain deleted still have good procoagulant activity. Eaton et al. disclose that an FVIII molecule that lacks 766 amino acids (797 to 1562) in the central B domain retains biological activity; in addition, FVIII with the B domain deleted is expressed in higher amounts than full-length molecules in mammal cells and exhibits a faster and higher activation rate than the full-length molecules (Eaton et al., Biochemistry, 1986, 25: 8343-8347). Other studies also show that deletion of major portion of the B domain (Ser743 to Gln1638) have no effect on the functional activity of FVIII, but significantly decrease the molecular weight (by 38%), and increase an expression level of FVIII in eukaryotic cells (Peters R T et al., J Thromb Haemost, 2013, 11(1): 132-141; Sandberg H et al., Semin Hematol, 2001, 38: 4-12).

Afstyla is a new recombinant single-chain human FVIII product and is the only single-chain coagulation factor product with a light chain and a heavy chain fused together, which is currently approved for the treatment of hemophilia A. Afstyla has higher molecular stability and longer duration of efficacy due to its strong affinity with von Willebrand Factor (vWF). In clinical studies, Afstyla demonstrates excellent hemostasis and bleeding prophylaxis effects in both a prophylaxis and an on-demand treatment, with relatively low dosage and very good safety in both administration schemes. Compared with a standard care drug Octocog alfa, Afstyla is more stable in protein configuration and has relatively durable efficacy, but still needs to be injected 2-3 times per week.

To extend the *in vivo* functional half-life of FVIII, in the existing art, FVIII is linked to a half-life extending moiety such as PEG, human serum albumin (HSA), transferrin, or IgG Fc. Currently, Novo Nordisk (N8-GP), Bayer (BAY94-9027), and Baxter (BAX 855) have developed long-lasting PEGylated FVIII products which have entered the stage of clinical studies. However, an additional step of chemically conjugating PEG to FVIII is added to a protein preparation process, reducing a final yield and increasing a preparation cost. On the other hand, data of pharmacokinetic studies shows that PEGylated FVIII has not achieved a significantly prolonged half-life (Tiede A et al., J Thromb Haemost, 2013, 11: 670-678; Turecek PL et al., Hamostaseologie, 2012, 32 Suppl 1: S29-38). WO2013106789 has disclosed a chimeric polypeptide (FVIIIFc) containing an FVIII moiety and an Fc moiety, where the terminal half-life of the chimeric FVIII polypeptide is extended to twice longer than that of rFVIII. In the prophylaxis, it is administered twice per week to maintain the level of FVIII activity at 1-3%.

CTP is a short peptide derived from the carboxyl terminus of human chorionic gonadotropin (hCG) β-subunit and contains multiple O-glycosylation sites. This negatively charged and highly sialylated peptide is covalently linked to the C-terminus of other proteins so as to be resistant to renal clearance, thereby extending the half-life of a target protein to which the CTP is linked *in vivo.* Some patent documents disclose that the CTP may be used as a half-life extending moiety included in a fusion protein. The present disclosure creatively uses a CTP polypeptide with multiple O-glycosylation sites as a part of a linker peptide for linking single-chain FVIII and an Fc fragment instead of being linked to the C-terminus to function as a fusion ligand. Since it has natural glycosylation sites, the CTP polypeptide can not only further prolong the half-life of the fusion protein and improve bioavailability but also cooperate with a conventional flexible GS flexible linker peptide to form a stable stereo conformation, thereby prompting single-chain FVIII and the Fc fragment to be independently folded to form a correct three-dimensional conformation, greatly reducing the steric hindrance effect of the fusion ligand Fc on single-chain FVIII, and enabling single-chain FVIII to maintain relatively high biological activity. In addition, the protective effect of CTP glycosyl side chains can reduce the susceptibility of the linker peptide to proteases.

In summary, although currently many studies provide effective schemes for preparing recombinant FVIII proteins, the heterologous number, structures and unstable thrombin activation of FVIII derivatives compared with full-length FVIII are still a serious problem. In addition, since a variety of B domain deleted FVIII are expressed as fusion proteins, the non-natural amino acid sequences may cause new immunogenicity when administrated. The stability of FVIII molecules is also critical for storage and clinical applications. In this case, it is desirable to develop activated, stable, and safe FVIII derivatives that can have the same thrombin activity, an increased yield, and a longer half-life so that the prophylaxis can be maintained by reducing the frequency of injections, helping to improve treatment effects.

### SUMMARY

The present disclosure provides an Fc fusion protein of a recombinant mutant single-chain coagulation factor VIII, which has an extended activity half-life *in vivo* and biological activity similar to that of recombinant FVIII. In addition, the present disclosure provides a method for efficiently and stably expressing the fusion protein. The fusion protein expressed by the method has the advantages of high yield, good stability in preparation and storage processes, and biological activity similar to that of recombinant FVIII on the market. The inventors of the present application have surprisingly found that the constructed recombinant mutant single-chain FVIII fusion protein has substantially enhanced stability, the fusion protein can avoid being cleaved by proteases in cells, and the obtained fusion protein exhibits better stability after being purified and good bioavailability when subcutaneously administered.

In a first aspect of the present disclosure, the present disclosure relates to a recombinant mutant single-chain coagulation factor VIII fusion protein. The fusion protein includes, sequentially from the N-terminus to the C-terminus, a single-chain human FVIII with a partially deleted B domain (scFVIII), a flexible peptide linker (L), at least one carboxyl-terminal peptide rigid unit of human chorionic gonadotropin β-subunit (hereinafter referred to as "CTP rigid unit" and expressed as (CTP)ₙ, wherein n is preferably 1, 2, 3, 4, or 5), and a half-life extending moiety (such as an immunoglobulin Fc fragment, albumin, transferrin, or PEG, preferably a human IgG Fc variant (expressed as vFc)). In some preferred embodiments of the present disclosure, the fusion protein is expressed as scFVIII-L-CTP-vFc.

The scFVIII lacks amino acids 765 to 1651 compared with the amino acid sequence of full-length human wild-type FVIII as shown in SEQ ID NO: 1. Specifically, the scFVIII has an amino acid sequence as shown in SEQ ID NO: 2.

The flexible peptide linker is preferably non-immunogenic, and produces a sufficient spatial distance between scFVIII and Fc to minimize steric hindrance therebetween. Preferably, used herein is a flexible peptide linker consisting of two or more amino acid residues which are selected from the following amino acids: Gly(G), Ser(S), Ala(A), and Thr(T).

More preferably, the flexible peptide linker includes G and S residues. The length of a linker peptide is very important for the activity of the fusion protein. For the present disclosure, the peptide linker may preferably have an amino acid sequence general formula formed by combining cyclic units (GS)a(GGS)b(GGGS)c(GGGGS)d, wherein a, b, c, and d are integers greater than or equal to 0 and a+b+c+d ≥ 1.

Specifically, in an embodiment of the present disclosure, the peptide linker may preferably include a following sequence:
(i) L1: GSGGGSGGGGSGGGGS;
(ii) L2: GSGGGGSGGGGSGGGGSGGGGSGGGGS;
(iii) L3: GGGGSGGGGSGGGGSGGGGS;
(iv) L4: GSGGGGSGGGGSGGGGSGGGGSGGGGSGGGGSGGGGS;
(v) L5: GGGSGGGSGGGSGGGSGGGS.

The CTP rigid unit is selected from a full-length sequence consisting of amino acids 113 to 145 at the carboxyl-terminus of human chorionic gonadotropin β-subunit or a fragment thereof. Specifically, the CTP rigid unit includes an amino acid sequence as shown in SEQ ID NO: 3 or a truncated sequence thereof. First, such CTP polypeptide that contains multiple glycosylation sites and naturally exists in human bodies is non-immunogenic. Secondly, compared to the flexible linker peptide that curls irregularly, a CTP rigid linker peptide containing multiple glycosylation sites can form a stable stereo conformation and promote the scFVIII and the Fc fragment to be independently folded to form a correct three-dimensional conformation without affecting their respective biological activity. In addition, the protective effect of CTP glycosyl side chains can reduce the susceptibility of the linker peptide to proteases.

Preferably, the CTP rigid unit includes at least two glycosylation sites. For example, in a preferred embodiment of the present disclosure, the CTP rigid unit includes two glycosylation sites. Exemplarily, the CTP rigid unit includes 10 amino acids at the N-terminus of SEQ ID NO: 3, that is, SSSS*KAPPPS*. Alternatively, the CTP rigid unit includes 14 amino acids at the C-terminus of SEQ ID NO: 3, that is, S*RLPGPS*DTPILPQ. For another example, in another embodiment, the CTP rigid unit includes three glycosylation sites. Exemplarily, the CTP rigid unit includes 16 amino acids at the N-terminus of SEQ ID NO: 3, that is, SSSS*KAPPPS*LPSPS*R. For another example, in some other embodiments, the CTP rigid unit includes four glycosylation sites. Exemplarily, the CTP rigid unit includes 28, 29, 30, 31, 32, or 33 amino acids from position 113, 114, 115, 116, 117, or 118 to position 145 of the human chorionic gonadotropin β-subunit. Specifically, the CTP rigid unit includes 28 amino acids at the N-terminus of SEQ ID NO: 3, that is, SSSS*KAPPPS*LPSPS*RLPGPS*DTPILPQ. Herein, * represents a glycosylation site. Each possibility represents an independent embodiment of the present disclosure.

In some other embodiments, the CTP rigid unit provided by the present disclosure is at least 70% homologous to a native CTP amino acid sequence. In some other embodiments, the CTP rigid unit provided by the present disclosure is at least 80% homologous to a native CTP amino acid sequence. In some other embodiments, the CTP rigid unit provided by the present disclosure is at least 90% homologous to a native CTP amino acid sequence. In some other embodiments, the CTP rigid unit provided by the present disclosure is at least 95% homologous to a native CTP amino acid sequence.

In a specific embodiment of the present disclosure, the CTP rigid unit may preferably include a following sequence:
(i) CTP¹: PRFQDSSSSKAPPPSLPSPSRLPGPSDTPILPQ;
(ii) CTP²: SSSSKAPPPSLPSPSRLPGPSDTPILPQ;
(iii) CTP³: SSSSKAPPPS;
(iv) CTP⁴: SRLPGPSDTPILPQ.

In some embodiments of the present disclosure, the fusion protein includes one CTP rigid unit described above. In some other embodiments of the present disclosure, the fusion protein includes two or more CTP rigid units described above, preferably 2, 3, 4, or 5 CTP rigid units described above.

The half-life extending moiety is preferably selected from an immunoglobulin IgG, IgM, or IgA Fc fragment, more preferably selected from an Fc fragment of human IgG1, IgG2, IgG3, or IgG4 or a variant thereof. Further, the human IgG Fc variant includes at least one amino acid modification in wild-type human IgG Fc, and the variant has a reduced effector function (ADCC and/or CDC effects) and/or enhanced binding affinity to a neonatal receptor FcRn. Further, the human IgG Fc variant may be selected from the following group:
(i) vFcγ₁: human IgG1 hinge region, CH2 region, and CH3 region containing Leu234Val, Leu235AIa, and Pro331 Ser mutations (such as an amino acid sequence as shown in SEQ ID NO: 4);
(ii) vFcγ₂₋₁: human IgG2 hinge region, CH2 region, and CH3 region containing Pro331 Ser mutation (such as an amino acid sequence as shown in SEQ ID NO: 5);
(iii) vFcγ₂₋₂: human IgG2 hinge region, CH2 region, and CH3 region containing Thr250Gln and Met428Leu mutations (such as an amino acid sequence as shown in SEQ ID NO: 6);
(iv) vFcγ₂₋₃: human IgG2 hinge region, CH2 region, and CH3 region containing Pro331 Ser, Thr250Gln, and Met428Leu mutations (such as an amino acid sequence as shown in SEQ ID NO: 7);
(v) vFcγ₄: human IgG4 hinge region, CH2 region, and CH3 region containing Ser228Pro and Leu235AIa mutations (such as an amino acid sequence as shown in SEQ ID NO: 8).

The IgG Fc variant provided by the present disclosure includes, but is not limited to, the above five variants in (i) to (v) and may also be a combination or superposition of mutation sites in two types of functional variants of IgG isotypes. For example, the variant in (iv) is a new IgG2 Fc combined variant obtained by superposing mutation sites in (ii) and (iii).

The Fc variant (vFc) in the fusion protein of the present disclosure contains the hinge region, CH2 region, and CH3 region of human IgG such as human IgG1, IgG2, or IgG4. The CH2 region contains amino acid mutations at positions 228, 234, 235, and 331 (determined by an EU counting system). It is believed that these amino acid mutations can reduce the effector function of Fc. Human IgG2 does not bind to FcyR but shows very low complement activity. An Fcy2 variant with Pro331Ser mutation should have lower complement activity than natural Fcγ2 and remains unbound to FcyR. IgG4 Fc is defective in activating complement cascade and its binding affinity to FcyR is about an order of magnitude lower than IgG1. An Fcy4 variant with Leu235Ala mutation should exhibit a minimum effector function compared with natural Fcy4. Fcy1 with Leu234Val, Leu235Ala, and Pro331 Ser mutations also exhibits reduced effector function compared with natural Fcy1. These Fc variants are all more suitable for the preparation of a FVIII fusion protein than natural human IgG Fc. Meanwhile, amino acid mutations at positions 250 and 428 (determined by an EU numbering system) increase the binding affinity of an Fc region to the neonatal receptor FcRn, further extending the half-life (Paul R et al., J Biol Chem, 2004, 279: 6213-6216). The combination or superposition of the above two types of functional variants gives a new combined variant which not only reduces the effector function but also extends the half-life. The Fc variant of the present disclosure includes, but is not limited to, mutations at the above several sites. Substitutions at other sites may also be introduced so that Fc has the reduced effector function and/or enhanced binding to an FcRn receptor without causing a decrease in the function/activity of the Fc variant or causing an adverse conformational change. For common mutation sites, see Shields RL et al., J Biol Chem, 2001, 276(9): 6591-604.

In a preferred embodiment of the present disclosure, the fusion protein has an amino acid sequence as shown by SEQ ID NO: 9.

In another aspect of the present disclosure, DNA for encoding the fusion protein described above is provided.

In a preferred embodiment of the present disclosure, the DNA for encoding the fusion protein has a sequence as shown in SEQ ID NO: 10.

In another aspect of the present disclosure, a vector is provided. The vector includes the DNA described above.

In another aspect of the present disclosure, a host cell is provided. The host cell includes the vector described above or has been transfected with the vector described above.

In a specific embodiment of the present disclosure, the host cell is a CHO-derived cell strain DXB-11.

In a fifth aspect of the present disclosure, a pharmaceutical composition is provided. The pharmaceutical composition includes a pharmaceutically acceptable carrier, excipient, or diluent and an effective amount of the fusion protein described above.

In another aspect of the present disclosure, a method for preparing or producing the fusion protein from a mammal cell line such as a CHO-derived cell line is provided, including the following steps:
(a) introducing DNA for encoding a fusion protein into a CHO cell to produce a CHO-derived cell line;
(b) screening in step (a) a high-yielding cell strain expressing more than 1 IU/10⁶ cells every 24 hours in a growth medium thereof;
(c) culturing the cell strain screened in step (b) to express the fusion protein;
(d) harvesting a fermentation broth obtained in step (c) and separating and purifying the fusion protein.

Further, the CHO-derived cell line in step (a) is DXB-11.

Further, cell culture in step (c) may be batch culture, perfusion culture, or fed-batch culture.

Further, in the step (d), the fusion protein is purified by four-step chromatography, namely, affinity chromatography, hydrophobic chromatography, anion exchange chromatography, and molecular sieve chromatography. Preferred conditions are further described in the present disclosure in conjunction with Example 5.

In a preferred embodiment of the present disclosure, the fusion protein prepared by the above method has activity of greater than 6000 IU/mg.

In a sixth aspect of the present disclosure, use of the fusion protein for preparing a medicament for the prevention or treatment of a hemorrhagic disease or event due to an FVIII deficiency or a functional deficiency is provided.

Further, the disease includes hemophilia A. The fusion protein of the present disclosure plays a role in controlling or preventing the occurrence of bleeding in the spontaneous bleeding event, surgical prophylaxis, perioperative management, or surgical treatment of a patient with hemophilia A.

The disclosed and/or described fusion protein and the preparation method thereof of the present disclosure have the following advantages:
1. The Fc fragment in the mutant single-chain FVIII fusion protein constructed in the present disclosure is non-lytic. That is, the complement and receptor binding domains of the Fc fragment are mutated to regulate the binding affinity of Fc to corresponding receptors so as to reduce or eliminate ADCC and CDC effects, such that the Fc fragment extends the *in vivo* half-life of active proteins without producing cytotoxicity.
2. The mutant single-chain FVIII fusion protein provided by the present disclosure includes a rigid CTP polypeptide with multiple glycosyl side chains. Compared to a flexible linker peptide such as (GGGGS)n that curls irregularly, the rigid CTP polypeptide can form a stable stereo conformation. This "separation" role promotes FVIII and the Fc fragment to be independently folded to form the correct three-dimensional conformation without affecting their respective biological activity. The CTP contains multiple O-modified oligosaccharides. The negatively charged and highly sialylated CTP can resist renal clearance and further extend the half-life of the fusion protein. On the other hand, the protective effect of CTP glycosyl side chains can reduce the susceptibility of the linker peptide to proteases so that the fusion protein is not easily degraded in the linker region.
3. The fusion protein of the present disclosure has good *in vitro* stability during fermentation, purification, and storage. After storage at room temperature i.e., 25 °C for seven days, the activity of the mutant single-chain FVIII fusion protein is significantly higher than that of a medicament of a recombinant double-chain FVIII Fc fusion protein where the protease cleavage sites of the fusion protein between Arg1648 and Glu1649 are not inactivated (encoded by a human wild-type FVIII full-length sequence i.e., SEQ ID NO: 1), and the activity loss is less than 20%.
4. The method for preparing the fusion protein provided by the present disclosure can achieve a high yield. An accumulated yield can reach at least 200 mg/L after incubation in a 300 mL shake flask for 14 days. The process can be scaled up to achieve large-scale industrial production.

### Detailed description of the disclosure

### Carboxyl-terminal peptide (CTP) of hCG-β

CTP is a short peptide derived from the carboxyl terminus of human chorionic gonadotropin (hCG) β-subunit. Four reproduction-related polypeptide hormones comprising follicle-stimulating hormone (FSH), luteinizing hormone (LH), thyroid-stimulating hormone (TSH), and human chorionic gonadotropin (hCG), contain the same α-subunit and their respective specific β-subunits. The *in vivo* half-life of hCG is significantly longer than those of the other three hormones, mainly due to the carboxyl-terminal peptide (CTP) specific to its β-subunit (Fares FA et al., Proc Natl Acad Sci USA, 1992, 89(10): 4304-4308). A natural CTP contains 37 amino acid residues, four O-glycosylation sites, and a terminal sialic acid residue. The negatively charged and highly sialylated CTP can resist renal clearance and thus extend the circulation half-life *in vivo* (Fares F A et al., Proc Natl Acad Sci USA, 1992, 89(10): 4304-4308). However, the present disclosure creatively links at least one CTP polypeptide with a flexible linker peptide with an appropriate length, which together serve as a linker peptide for linking FVIII to a half-life extending moiety (such as an immunoglobulin Fc fragment).

The present disclosure has found that the addition of the CTP between FVIII and an Fc variant is equivalent to the addition of a rigid linker peptide. This, on one hand, ensures that FVIII fused at the N-terminus does not affect a binding site of the Fc variant to FcRn so that the half-life is not affected. In addition, the binding site of protein A in Fc is important for purification in a preparation process. Linking of CTP ensures that FVIII fused at the N-terminus does not "cover" its binding site with protein A so that cheaper and more suitable fillers can be selected to purify the fusion protein, reducing a purification cost. On the other hand, the addition of the CTP enables the Fc fragment of 25 kDa not to interfere with the correct folding of FVIII fused at the N-terminus so that the biological activity/function of FVIII is not decreased or lost. Compared to a flexible linker peptide such as (GGGGS)n that curls irregularly, the rigid CTP polypeptide with multiple glycosyl side chains can form a stable stereo conformation. This "separation" role promotes FVIII and the Fc fragment to be independently folded to form the correct three-dimensional conformation without affecting their respective biological activity. On yet another hand, the protective effect of CTP glycosyl side chains can reduce the susceptibility of the linker peptide to proteases so that the fusion protein is not easily degraded in the linker region.

### IgG Fc variant

### Non-lytic Fc variant

The Fc element is derived from an Fc fragment in a constant region of immunoglobulin IgG and plays an important role in the immune defense against a pathogen. The effector function of IgG mediated by Fc is performed through the following two mechanisms: (1) Fc binds to Fc receptors (FcyRs) on cell surface, so that the pathogen is digested by phagocytosis or lysis or by killer cells via an antibody-dependent cytotoxicity (ADCC) pathway, or (2) Fc binds to the C1q of a first complement component C1 and triggers a complement-dependent cytotoxicity (CDC) pathway to lyse the pathogen. Among four human IgG subtypes, IgG1 and IgG3 can effectively bind to FcyRs, IgG4 has lower binding affinity to FcyRs, and IgG2 has too low binding affinity to FcyRs to be determined. Therefore, human IgG2 has almost no ADCC effect. In addition, human IgG1 and IgG3 can effectively bind to C1q to activate complement cascade. Human IgG2 weakly binds to C1q and IgG4 does not bind to C1q (Jefferis R et al., Immunol Rev, 1998, 163: 59-76). Therefore, human IgG2 has a weak CDC effect. Apparently, none of the natural IgG subtypes is ideal for constructing a FVIII-Fc fusion protein. To obtain non-lytic Fc with no effector function, the most effective method is to mutate the complement and receptor binding domains of the Fc fragment to regulate the binding affinity of Fc to corresponding receptors, reduce or eliminate ADCC and CDC effects, and retain the biological activity of the functional protein and the extended half-life *in vivo* of the Fc fragment without producing cytotoxicity. For more mutation sites included in the non-lytic Fc variant, see Shields RL et al., J Biol Chem, 2001, 276(9): 6591-604 or Chinese Patent No. CN 201280031137.2.

### Fc variant with enhanced binding affinity to neonatal receptor (FcRn)

The plasma half-life of IgG depends on its binding to FcRn, where IgG generally binds at a pH of 6.0 and dissociates at a pH of 7.4 (the pH of plasma). Through studies on the binding sites, binding sites of IgG to FcRn have been modified to increase the binding capacity at pH 6.0. It has been proved that mutations of some residues of a human Fcγ domain important for the binding to FcRn can increase serum half-life. It has been reported that mutations at T250, M252, S254, T256, V308, E380, M428, and N434 can increase or decrease the binding affinity to FcRn (Roopenian et al., Nat. Rview Immunology, 7: 715-725, 2007). Korean Patent No. KR 10-1027427 discloses trastuzumab (Herceptin, Genentech) variants with enhanced binding affinity to FcRn, where these variants include one or more amino acid modifications selected from 257C, 257M, 257L, 257N, 257Y, 279Q, 279Y, 308F, and 308Y. Korean Patent Publication No. KR 2010-0099179 provides bevacizumab (Avastin, Genentech) variants, where these variants exhibit an increased *in vivo* half-life by containing amino acid modifications at N434S, M252Y/M428L, M252Y/N434S, and M428L/N434S. In addition, Hinton et al. have found that T250Q and M428L mutants increase the binding to FcRn by 3 and 7-fold, respectively. When two site are mutated simultaneously, the binding is increased 28-fold. In rhesus monkeys, M428L or T250QM/428 L mutant exhibits the plasma half-life increased 2-fold (Paul R. Hinton et al., J Immunol, 2006, 176: 346-356). For more mutation sites included in the Fc variant with the enhanced binding affinity to the neonatal receptor (FcRn), see Chinese Patent No. CN201280066663.2. In addition, studies show that T250Q/M428L mutation in the Fc fragment of five humanized antibodies improves the interaction between Fc and FcRn and also show that, in subsequent *in vivo* pharmacokinetic assays, the Fc mutant antibodies, when administrated by subcutaneous injection, have improved pharmacokinetic parameters such as increased *in vivo* exposure, reduced clearance, and increased subcutaneous bioavailability compared with a wild-type antibody (Datta-Mannan A et al., MAbs. Taylor&Francis, 2012, 4: 267-273).

### Fusion protein and preparation method thereof

Genes of the fusion protein of the present disclosure are prepared by an artificial synthesis method with optimized codons. According to a nucleotide sequence in the present disclosure, those skilled in the art may conveniently prepare encoding nucleic acids of the present disclosure by various known methods. These methods are not limited to an artificial synthesis or traditional subcloning, etc. For specific methods, see Molecular Cloning: A Laboratory Manual by J. Sambrook. As an embodiment of the present disclosure, an encoding nucleic acid sequence of the present disclosure is constructed by synthesizing segments of the nucleotide sequence and subcloning.

The present disclosure further provides an expression vector of mammal cells, which includes a sequence for encoding the fusion protein of the present disclosure and an expression regulatory sequence operatively linked thereto. The term "operatively linked" or "operably linked" refers to such a case where a certain portion of a linear DNA sequence can regulate or control the activity of other portions of the same linear DNA sequence. For example, if a promoter controls the transcription of a sequence, the promoter is operably linked to the encoding sequence.

The expression vector of mammal cells may use commercially available vectors such as, but not limited to, pcDNA3, pIRES, pDR, pBK, and pSPORT that can be used for the expression in a eukaryotic cell system. Those skilled in the art may also select an appropriate expression vector according to the host cell.

According to the restriction map of the known empty expression vector, those skilled in the art may insert the encoding sequence of the fusion protein of the present disclosure into appropriate restriction sites through restriction enzyme cleavage and splicing according to a conventional method to obtain a recombinant expression vector of the present disclosure.

The present disclosure further provides a host cell that expresses the fusion protein of the present disclosure and contains the encoding sequence of the fusion protein of the present disclosure. The host cells are preferably eukaryotic cells such as, but not limited to, CHO cells, COS cells, 293 cells, and RSF cells. As a preferred embodiment of the present disclosure, the cells are CHO cells which can preferably express the fusion protein of the present disclosure so that the fusion protein with good activity and stability can be obtained.

The present disclosure further provides a method for preparing the fusion protein of the present disclosure using a recombinant DNA technology, comprising the following steps:
(1) providing a nucleic acid sequence for encoding the fusion protein;
(2) inserting the nucleic acid sequence in (1) into an appropriate expression vector to obtain a recombinant expression vector;
(3) introducing the recombinant expression vector in (2) into an appropriate host cell;
(4) culturing the transfected host cell under conditions suitable for expression; and
(5) collecting a supernatant and purifying a fusion protein product.

The encoding sequence may be introduced into the host cell using various technologies known in the art, such as, but not limited to, calcium phosphate precipitation, lipofection, electroporation, microinjection, viral infection, and alkali metal ions.

For the culture and expression of host cells, see Olander RM et al., Dev Biol Stand, 1996, 86: 338. Cells and residues in a suspension may be removed by centrifugation to collect the supernatant.

The fusion protein prepared above may be purified to obtain substantially homogeneous properties such as a single or particular band on SDS-PAGE electrophoresis. First, the expression supernatant is concentrated and the concentrated solution may be further purified through gel chromatography or ion exchange chromatography such as anion exchange chromatography or cation exchange chromatography. The gel matrix may be a medium commonly used for protein purification, such as agarose, dextran, and polyamide. Q- or SP-groups are relatively ideal ion exchange groups. Finally, the purified product may be further refined by methods such as hydroxyapatite adsorption chromatography, metal chelation chromatography, hydrophobic interaction chromatography, and reversed-phase high-performance liquid chromatography. In all of the above purification steps, different combinations may be used to achieve substantially uniform protein purity. The expressed fusion protein may also be purified using an affinity chromatography column containing an antibody, receptor, or ligand specific for the fusion protein. According to the properties of the affinity column used, fusion polypeptides that bind to the affinity column may be eluted by conventional methods such as a high salt buffer and a pH change.

### Pharmaceutical composition

The present disclosure further provides a pharmaceutical composition containing an effective dose of the fusion protein of the present disclosure and a pharmaceutically acceptable carrier. Generally, an effective amount of the fusion protein of the present disclosure may be formulated in a non-toxic, inert and pharmaceutically acceptable aqueous carrier medium, where the pH is generally about 5-8, preferably, the pH is about 6-8. The term "effective amount" or "effective dose" refers to an amount that can be functional or active to and acceptable by humans and/or animals. "Pharmaceutically acceptable" ingredients are those that are applicable to humans and/or mammals without excessive adverse side effects (such as toxicity, irritation, and allergic reactions), that is, substances with a reasonable benefit/risk ratio. The term "pharmaceutically acceptable carrier" refers to a carrier for the administration of a therapeutic agent, which includes various excipients and diluents.

Pharmaceutically acceptable carriers include (but not limited to): saline, buffer, glucose, water, glycerol, ethanol, and combinations thereof. Generally, a pharmaceutical preparation should match with the mode of administration. The pharmaceutical composition of the present disclosure may be made in the form of injections, for example, prepared by conventional methods using normal saline or aqueous solutions containing glucose and other adjuvants. The pharmaceutical composition is preferably produced under a sterile condition. The amount of active ingredient administered is a therapeutically effective amount. The pharmaceutical preparation of the present disclosure may also be made into a sustained-release preparation.

The effective amount of the fusion protein of the present disclosure may vary depending on the mode of administration and the severity of a disease to be treated. The selection of a preferred effective amount may be determined by those of ordinary skill in the art according to various factors (for example, through clinical trials). The factors include, but are not limited to, the pharmacokinetic parameters of the fusion protein such as bioavailability, metabolism, and the half-life, etc; the severity of the disease to be treated, the weight of a patient, the immune status of the patient, and a path of administration, etc.

### BRIEF DESCRIPTION OF DRAWINGS

FIG. 1 is a diagram of SEC-HPLC spectrogram of a purified fusion protein SS-F8.
FIG. 2 is a diagram of SDS-PAGE electrophoresis of a purified fusion protein SS-F8.
FIG. 3 shows an effect of a fusion protein SS-F8 on bleeding time of saphenous arteries in SD rats.
FIG. 4 shows an effect of a fusion protein SS-F8 on APTT of plasma in SD rats.
FIG. 5 is a statistics diagram of bleeding volume in mice, where **p* < *0.05, ****p < 0.0001.*
FIG. 6 is a statistics diagram of bleeding time in mice, where *ns p > 0.05, ****p < 0.0001.*

### DETAILED DESCRIPTION

### Example 1. Construction of expression plasmids for encoding mutant single-chain FVIII fusion proteins

Gene sequences for encoding an FVIII leader peptide, FVIII protein with a partially deleted B domain, a flexible peptide linker, a CTP rigid unit, and a human IgG vFc variant were artificially optimized codons that CHO cells prefer, and were artificially synthesized. The synthesized full-length DNA fragment of the fusion protein included one restriction endonuclease site, Spel and EcoRI, at 5' and 3' ends, respectively. The full-length DNA fragment was inserted between corresponding restriction sites of pUC57 transfer vector and its sequence was verified through DNA sequencing.

The full-length gene fragment obtained above of the fusion protein was transferred from the intermediate vector to a self-made expression plasmid PXY1A1M between the corresponding restriction sites to obtain a plasmid that expressed high level of the fusion protein. The PXY1A1M plasmid included, but was not limited to, the following important expression elements: (1) a human cytomegalovirus early promoter, and an enhancer required for exogenous high expression in mammal cells; (2) a double screening marker with kanamycin resistance in bacteria and G418 resistance in mammal cells; (3) murine dihydrofolate reductase (DHFR) gene expression cassette. Methotrexate (MTX) can co-amplify fusion genes and DHFR genes when a host cell is deficient in the DHFR genes (see US 4,399,216). The expression plasmid of the fusion protein was transfected into a mammal host cell line. To achieve stable and high-level expression, a preferred host cell line is a DHFR enzyme deficient CHO-cell (see US Patent No. 4,818,679). After two days of transfection, the medium was replaced with a screening medium containing 0.6 mg/mL G418, and the cells were seeded in a 96-well plate at a certain concentration (5000-10000 viable cells/well) and cultured for 10-14 days until large discrete cell clones appeared. Transfectants resistant to selected drugs were screened through ELISA analysis method. Wells with high-level fusion proteins were subcloned through the extreme dilution of the 96-well culture plate.

A series of mutant single-chain FVIII fusion proteins were constructed in the present disclosure, which contained peptide linkers of different lengths, CTP rigid units of different compositions, and several different subtypes of IgG Fc variants (vFc), to verify the effect of linker peptides and Fc variants on the activity of the mutant single-chain FVIII fusion proteins. Details are shown in Table 1. The amino acid sequences of each component are found in SEQUENCE LISTING.

**Table 1 Compositions of various single-chain FVIII proteins**

| Code | Compositions of a Series of FVIII Fusion Proteins (from the N-terminus to C-terminus) |
|---|---|
| SS-F4 | scFVIII-L3-CTP¹-vFcγ₁ |
| SS-F5 | scFVIII-L5-CTP⁴-vFcγ₄ |
| SS-F6 | scFVIII-L1-CTP³-CTP³-vFcγ₂₋₂ |
| SS-F7 | scFVIII-L4-CTP³-vFcγ₂₋₁ |
| SS-F8 | scFVIII-L2-CTP²-vFcγ₂₋₃ |
| SS-F9 | scFVIII-L2-vFcγ₂₋₃-CTP² |
| SS-F10 | scFVIII-L1-vFcγ₂₋₃ |
| SS-F11 | scFVIII-L4-vFcγ₂₋₃ |

### Example 2. Screening of a stably transfected cell line with high expression of fusion protein

The above expression plasmid of a fusion protein was transfected into a mammal host cell line to express the mutant single-chain FVIII fusion protein. To maintain stable and high-level expression, a preferred host cell is a DHFR deficient CHO-cell (see US Patent No. 4,818,679). A preferred transfection method is electroporation. Other methods may be used, such as calcium phosphate co-precipitation, lipofection, and microinjection. The electroporation method used Gene Pulser Electroporator (Bio-Rad Laboratories) with a voltage of 300 V and a capacitance of 1050 µFd, and 50 µg of Pvul linearized expression plasmids was added to 2 to 3 × 10⁷ cells placed in a cuvette, and the cells after electroporation were transferred to a shake flask containing 30 mL of growth medium. After two days of transfection, the medium was replaced with a growth medium containing 0.6 mg/mL G418, and the cells were seeded in a 96-well plate at a certain concentration and cultured for 10-12 days until large discrete cell clones appeared. Transfectants resistant to selected drugs were screened through ELISA analysis method against human IgG Fc. Wells with high-level expression of fusion proteins were subcloned through extreme dilution.

To achieve the high-level expression of fusion proteins, DHFR gene suppressed by MTX should be used for co-amplification. The transfected fusion protein genes were co-amplified with the DHFR gene in growth media containing MTX with increased concentrations. Positive subclones that expressed DHFR were subjected to limiting dilution, and the pressure was increased gradually to screen out transfectants that could grow in media with MTX of up to 6 µM. The secretion rates of the transfectants were determined and cell lines with high exogenous protein expression were screened. Cell lines with a secretion rate greater than about 1 (preferably about three) IU/10⁶ (million) cells/24 h were adaptively suspended using a serum-free medium, and the fusion proteins were then purified using a conditioned medium.

### Example 3. Production of fusion proteins

The high-yield cell strains preferably obtained in Example 2 were first subjected to serum-free acclimation and culture in a petri dish, and then transferred to a shake flask for suspension acclimation and culture. After the cells were acclimated to these culture conditions, fed-batch culture was performed in a 300 mL shake flask or perfusion culture was simulated by changing media daily. A CHO-derived cell strain that was screened in Example 2 for producing fusion protein SS-F8 was subjected to fed-batch culture for 14 days in a 300 mL shake flask, where a cumulative yield of expressed recombinant fusion proteins reached 200 mg/L and a maximum viable cell density reached 15 × 10⁶/mL. To obtain more fusion proteins, a 1000 mL shake flask may also be used for culture. In another culture method, the above-mentioned CHO-derived cell strain was cultured in a 100 mL shake flask with media changed daily, where a daily cumulative yield of expressed recombinant fusion proteins was about 20 mg/L and a maximum viable cell density in the shake flask reached 30 × 10⁶/mL. The biological activity of the recombinant fusion proteins produced by the above two methods was determined to be comparable.

### Example 4. Purification and quantization of fusion proteins

Fusion protein SS-F8 was mainly purified through four-step chromatography in the present disclosure. The four-step chromatography was respectively affinity chromatography, anion exchange chromatography, hydrophobic chromatography, and molecular sieve chromatography. (the protein purifier used in this example was AKTA pure 25 M from GE, U.S; and reagents used in this example were purchased from Sinopharm Chemical Reagent Co., Ltd and had purity at an analytical grade).

In a first step, affinity chromatography was performed. Sample capture, concentration and the removal of partial pollutants were performed using VIII Select Affinity Chromatography Media from GE. First, the chromatography column was equilibrated with 3-5 column volumes (CVs) of an equilibration buffer containing 10 mM of HEPES, 150 mM of NaCl, 25 mM of CaCl₂, and 0.05% Tween-80 and with a pH of 6.8-7.2 at a linear flow rate of 50-100 cm/h. The clarified fermentation broth was loaded at a linear flow rate of 50-100 cm/h with a load not higher than 50000 IU/mL. After loading, the chromatography column was equilibrated with 3-5 column volumes (CVs) of an equilibration buffer containing 10 mM of HEPES, 150 mM of NaCl, 5 mM of CaCl₂, and 0.05% Tween-80 and with a pH of 6.8-7.2 at a linear flow rate of 50-100 cm/h, and unbound components were rinsed. The chromatography column was rinsed with 3-5 column volumes of a decontamination buffer 1 containing 10 mM of HEPES, 1 M of NaCl, 25 mM of CaCl₂, and 0.05% Tween-80 and with a pH of 6.8-7.2 at a linear flow rate of 50-100 cm/h to remove partial pollutants. The chromatography column was equilibrated with 3-5 column volumes (CVs) of an equilibration buffer containing 10 mM of HEPES, 150 mM of NaCl, 25 mM of CaCl₂, and 0.05% Tween-80 and with a pH of 6.8-7.2 at a linear flow rate of 50-100 cm/h. Then the target product was eluted at a linear flow rate not higher than 50 cm/h using an elution buffer containing 20 mM of His-HCI, 25 mM of CaCl₂, 0.02% Tween-80, and 45% propylene glycol and with a pH of 6.8-7.2 and target peaks were collected.

In a second step, anion exchange chromatography was performed. Intermediate purification was conducted by using Q-HP of Bestchrom or other commercially available anion exchange chromatography media (such as Q HP of GE, Toyopearl GigaCap Q-650 of TOSOH, DEAE Beads 6FF of Smart-Lifesciences, Generik MC-Q of Sepax Technologies, Inc, Fractogel EMD TMAE of Merck, and Q Ceramic HyperD F of Pall) to separate structural variants and further remove pollutants such as HCP and DNA. First, the chromatography column was rinsed with 3-5 column volumes (CVs) of an equilibration buffer containing 20 mM of His-HCI, 100 mM of NaCl, 10 mM of CaCl₂, and 0.02% Tween-80 and with a pH of 7.0-7.5 at a linear flow rate of 50-100 cm/h. The target proteins separated through affinity chromatography in the first step were diluted 5-10 times to reduce the concentration of organics. Then samples were loaded with the load being controlled within 5000-10000 IU/mL. After loading, the chromatography column was rinsed with 3-5 column volumes (CVs) of an equilibration buffer containing 20 mM of His-HCI, 100 mM of NaCl, 10 mM of CaCl₂, and 0.02% Tween-80 and with a pH of 7.0-7.5 at a linear flow rate of 50-100 cm/h. Then, the chromatography column was rinsed with 3-5 column volumes (CVs) of a wash buffer containing 20 mM of His-HCI, 500 mM of NaCl, 10 mM of CaCl₂, and 0.02% Tween-80 and with a pH of 7.0-7.5 at a linear flow rate of 50-100 cm/h to remove partial impurity proteins. The chromatography column was then eluted with gradient salt concentrations using an elution buffer containing 20 mM of His-HCI, 1 M of NaCl, 10 mM of CaCl₂, and 0.02% Tween-80 and with a pH of 7.0-7.5, where the condition was elution buffer from 50% and 60%, 3-5 column volumes (CVs), and a linear flow rate not higher than 50 cm/h. Eluted fractions were collected. The samples were sent for detecting protein content, SEC-HPLC, activity, and HCP content separately. A protein concentration and protein activity were determined.

In a third step, hydrophobic chromatography was performed. Intermediate purification was conducted by using Butyl HP of Bestchrom or other commercially available hydrophobic chromatography media (such as Butyl HP of GE, Toyopearl Butyl-650 of TOSOH, Butyl Beads 4FF of Smart-Lifesciences, Generik MC30-HIC Butyl of Sepax Technologies, Inc, and Fractogel EMD Propyl of Merck) to reduce the content of polymers. The eluate obtained after anion exchange chromatography in the second step still contained a certain proportion of polymers. Because of various reasons for the formation of the polymers such as polymerization with unchanged structures and polymerization with changed structures, the biological activity of the polymers varies greatly and thus causes great interference to the analysis of biological activity. When the target proteins are polymerized, the polymers and monomers differ in property including charge characteristics and hydrophobicity. Polymers and monomers were separated using differences in hydrophobicity. Since the final step of purification would be molecular sieve chromatography, purification was performed using Butyl HP to remove partial polymers to a content less than 10%. First, the chromatography column was equilibrated with 3-5 column volumes (CVs) of an equilibration buffer containing 20 mM of His-HCI, 1.5 M of NaCl, 5 mM of CaCl₂, and 0.02% Tween-80 and with a pH of 6.8-7.2 at a linear flow rate of 50-100 cm/h. The conductivity of the target proteins separated through anion exchange chromatography in the second step was adjusted using a buffer containing 2 M of (NH₄)₂SO₄. Then samples were loaded with a load being controlled lower than 20000 IU/mL. After loading, the chromatography column was rinsed with 3-5 column volumes (CVs) of an equilibration buffer containing 20 mM of His-HCI, 1.5 M of NaCl, 5 mM of CaCl₂, and 0.02% Tween-80 and with a pH of 6.8-7.2 at a linear flow rate of 50-100 cm/h. Then, the chromatography column was rinsed with 3-5 column volumes (CVs) of a wash buffer containing 20 mM of His-HCI, 1.5 M of NaCl, 5 mM of CaCl₂, and 0.02% Tween-80 and with a pH of 6.8-7.2 at a linear flow rate of 50-100 cm/h to remove partial polymers. Finally, the target proteins were eluted respectively with 3-5 column volumes (CVs) of 20%, 40%, and 100% of an elution buffer containing 20 mM of His-HCI, 5 mM of CaCl₂, 0.02% Tween-80, and 50% ethylene glycol and with a pH of 6.8-7.2 at a linear flow rate not higher than 60 cm/h. Eluted fractions were collected and sent for detecting SEC-HPLC separately. Target components with the percentage of monomers being greater than 90% were combined for chromatography in the next step.

In a fourth step, molecular sieve chromatography was performed. Separation was conducted by using superdex 200 of GE or other commercially available molecular sieve media (such as Chromdex 200 prep grade of Bestchrom) to reduce the content of polymers to be lower than 5% and further reduce the content of key pollutans. The chromatography column was rinsed with 2 column volumes (CVs) of an equilibration buffer containing 10 mM of His-HCI, 150 mM of NaCl, 2 mM of CaCl₂, 10 mM of sucrose, and 0.02% Tween-80 and with a pH of 6.8-7.2 at a linear flow rate of 20-40 cm/h. The load was not higher than 3% of the column volume and the samples were rinsed at a linear flow rate of 20 cm/h. Elution components were sequentially collected and combined for SEC detection.

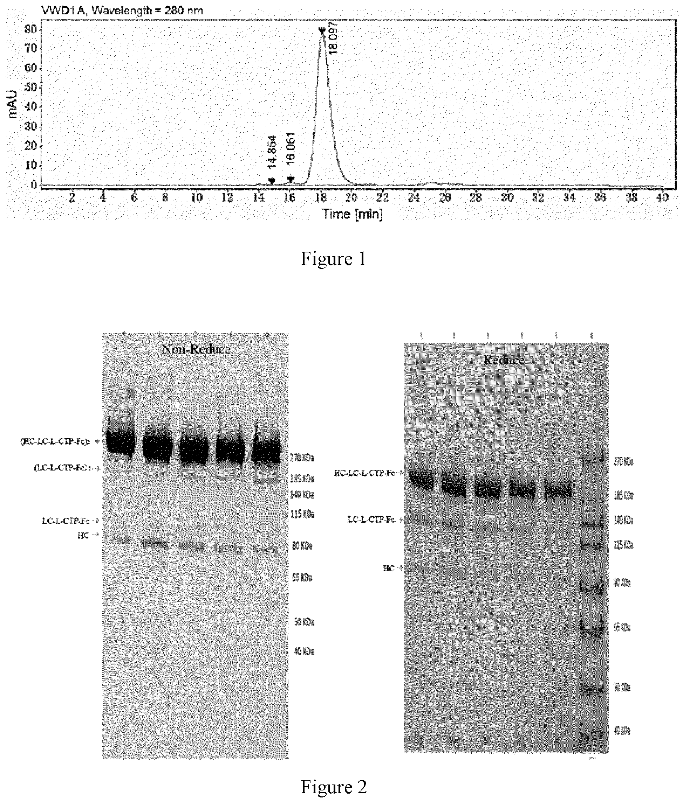

The SEC-HPLC purity result and SDS-PAGE electrophoresis result of the samples are shown in FIG. 1 and FIG. 2 respectively. The SEC-HPLC result shows that the purity of main peaks of the purified fusion proteins was higher than 98.0%. The SDS-PAGE electrophoretic pattern was as expected. The non-reducing electrophoresis included bands of an unprocessed fusion protein (about 390 kDa), a (LC-L-CTP-Fc)₂ dimer fragment (about 210 kDa) formed after shedding of two heavy-chain fragments upon cleavage at another common cleavage site E720, an LC-L-CTP-Fc single-chain fragment (about 100 kDa) and an HC fragment (about 90 kDa) . After reduction, clear HC-LC-L-CTP-Fc (about 190 kDa), LC-L-CTP-Fc (about 105 kDa) and HC (about 90 kDa) single-chain bands were obtained. The specific activity of the obtained protein components was 6000-8000 IU/mg.

### Example 5. Indirect determination of the activity of the fusion protein in vitro by a chromogenic substrate method

The activity of the mutant single-chain FVIII fusion protein may be determined by the chromogenic substrate method. In this example, the activity was determined using Biophen FVIII:C kit (HYPHEN BioMed, Ref. 221402) based on the following principle: when activated by thrombin, FVIII:C binds to FIXa in the presence of phospholipids and calcium ions to form an enzyme complex which in turn can activate factor X to transform into its active form Xa. The active factor Xa can cause the cleavage of its specific chromogenic substrate (SXa-11) to release chromogenic group pNA. The activity of FXa directly proportional to the amount of pNA may be obtained by determining the amount of produced pNA at 405 nm. In this system, the contents of factor IXa and factor X are certain and excessive, and the activity of FXa is only directly related to the content of FVIIIa. The specific activity of the mutant single-chain FVIII fusion protein determined by this method was about 6000-8000 IU/mg.

### Example 6. Studies on the stability of the purified fusion protein

To further verify the stability of the mutant single-chain FVIII fusion protein at room temperature of 25°C, the mutant single-chain FVIII fusion protein was stored at room temperature of 25°C for several days to investigate the effect on the activity of the fusion protein.

A medicine stability testing chamber (purchased from Shanghai Yiheng Scientific Instrument instruments Co., Ltd.) was used to set a test temperature of 25°C and a humidity of 75%. Eight copies of SS-F8 and double-chain factor VIII reference drug DS-F8 (a fusion protein retaining the protease cleavage site between Arg1648 and Glu1649 of human wild-type FVIII and having an amino acid sequence as shown in SEQ ID NO: 11) that were diluted to the same concentration, 200 µL for each copy, were stored in the medicine stability testing chamber. One copy of SS-F8 and one copy of DS-F8 were taken and the activity of the fusion proteins was assayed according to the method in Example 5 and recorded as activity values on the first day (d1). Then, the activity of the mutant single-chain FVIII fusion protein was assayed separately on d3, d5, d7 and d14 after the fusion proteins were placed at room temperature (temperature: 25°C, humidity: 75%). The results show that the protein activity of SS-F8 only decreased by about 10% and the protein activity of DS-F8 decreased by more than 25% after 5 days at room temperature; the activity of DS-F8 decreased at a significantly higher rate than that of SS-F8 after 7 days at room temperature; and the protein activity of SS-F8 still remained 80% after 7 days. It can be seen that fusion protein SS-F8 has significantly better stability than DS-F8.

### Example 7. Pharmacodynamic study on the fusion protein

Blood coagulation is in fact enzymatic reactions of a series of coagulation factors. The whole coagulation process is divided into three stages: the first stage is the formation of blood thromboplastin; the second stage is the formation of thrombin; and the third stage is the formation of fibrin, where FVIII and FIX are endogenous coagulation factors and FVII is an exogenous coagulation factor. Bleeding time is the time from natural bleeding to natural hemostasis after skin capillaries are punctured. The procoagulant effect of the fusion protein was detected by observing the effect of the mutant single-chain FVIII fusion protein on the bleeding time of saphenous arteries in SD rats.

Seven-week-old SD rats (purchased from Shanghai SLAC Laboratory Animal Co., Ltd) were selected and randomly divided into two groups. The administration group was administered intravenously with SS-F8 at a single dosage of 200 IU/rat and the control group was administered with the same volume of normal saline. Three hours after administration, rats were induced to be anesthetized. After the surgical position was disinfected, the skin was cut from about 1 cm above the medial malleolus through the knee joint to the artery at leg root; subcutaneous tissues were separated from the supra-vascular protective membrane; and venous vessels, arterial vessels and nerves were exposed in sequence. The saphenous artery and its branches were found at the position of the knee joint. Muscle positions within 5 mm*1 mm near the venous vessels were sharp-dissected with microscopic straight forceps, and the veins, arteries and nerves were lifted from the medial side. The saphenous vein and the saphenous artery were cut with a microscissor and the nerves were not touched or injured. The initial bleeding time was the time when the blood gushed out and recorded as t1. The bleeding site was observed every 30 seconds until no bleeding was seen. The time was recorded as hemostasis time t2. The bleeding time was recorded through a calculation of t2-t1. A statistical analysis was performed on experimental data that was expressed as means ± a standard deviation (SD). If the data follows normal distribution, SPSS *18.0 software* was used for one-way analysis of variance or student's-test. In the case of non-normal distribution, a non-parametric test such as a Kruskal-wallis test or a Mann-whitney test was used, where P ≤ 0.05 denotes a significant difference and P ≤ 0.01 denotes a very significant difference.

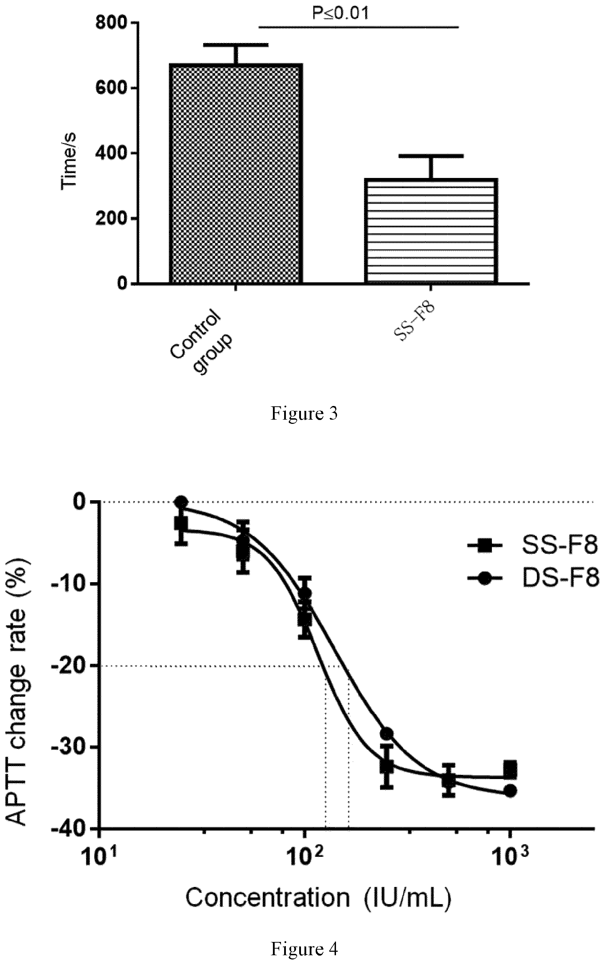

The experimental results are shown in FIG. 3. After prophylaxis administration, the bleeding time of the SS-F8 group was significantly different from that of the normal saline group in statistics after the bleeding of the saphenous artery caused by a surgery in SD rats. At the dosage of 200 IU/rat, the prophylactic administration of SS-F8 has a significant hemostatic effect on the bleeding model.

### Example 8. Direct determination of the biological activity of the fusion protein by a coagulation method

The *in vitro* anticoagulant effect of SS-F8 on SD rat plasma was observed by determining activated partial thromboplastin time (APTT).

Seven-week-old SD rats (purchased from Shanghai SLAC Laboratory Animal Co., Ltd) were selected and randomly divided into 12 groups. After induced to be anesthetized, the rats were cut along the median line of the abdomen with a scalpel under continuous anesthesia, and 10-12 mL of blood was taken from the abdominal aorta. The above blood samples were centrifuged at 20°C and 1500 rpm for 30 min, and supernatant plasma was separated and added to labeled 1.5 mL centrifuge tubes separately. The whole plasma and test drugs SS-F8 and DS-F8 were prepared at a volume ratio of 6: 1 into test samples with concentrations of 25-1000 IU/mL respectively. In a control group, a diluent was added at a volume ratio of 6:1. The APTT values of the above samples were tested with a fully automated coagulator (CS-2000i, Sysmex). The change rate of APTT was calculated according to the following formula: the change rate of APTT = (the APTT value of the administration group - the APTT value of the control group)/the APTT value of the control group. The experimental data was expressed as means ± a standard deviation (SD). If the data follows normal distribution, SPSS *18.0 software* was used for one-way analysis of variance or student's-test. In the case of non-normal distribution, a non-parametric test such as a Kruskal-wallis test or a Mann-whitney test was used, where P ≤ 0.05 denotes a significant difference and P ≤ 0.01 denotes a very significant difference.

It can be seen from FIG. 4 that the test drugs SS-F8 and DS-F8 had anticoagulant effect on the plasma of normal rats; the APTT of the test drugs DS-F8 and SS-F8 at a high concentration of 1000 IU/mL decreased by 33.65% and 31.86%, respectively, compared with the vehicle control group; the EC₅₀ values of DS-F8 and SS-F8 were 139.4 IU/mL and 115.6 IU/mL, respectively; and the EC₅₀ value of SS-F8 was lower than that of DS-F8, which suggests a lower dosage. With an increase in the concentration of the test drugs, the APTT was significantly reduced and there was a dose-effect relationship.

### Example 9. Hemostatic effect of the fusion protein on acute hemorrhage in mice with hemophilia A

The acute hemostatic effect of SS-F8 in mice with hemophilia A was evaluated through a tail clip bleeding model of VIII factor gene knockout homozygous hemophilia A (HA). Male HA mice at the age of 6-7 weeks (from Shanghai Model Organisms Center, Inc.) were selected, adaptively fed for one week, and randomly divided into two groups: a HA mouse blank vehicle control group and an SS-F8 administration group. Male C57BL/6 mice (purchased from Shanghai SLAC Laboratory Animal Co., Ltd) were selected as a normal control group. Mice were anesthetized through the intraperitoneal injection of 1% sodium pentobarbital (Merck & Co.) at the dosage of 7.5 mL/kg. The C57BL/6 mouse normal control group and the HA mouse blank vehicle control group were administered intravenously via tail veins with a vehicle at the dosage of 10 mL/kg, and the administration group was administered intravenously via tail veins with SS-F8 at the dosage of 112 IU/kg. The tails of the mice were clipped 15 mm from the ends of the tails using a scalpel blade 15 minutes after the administration. The wounds were quickly immersed in 13 mL of normal saline preheated to 37°C. The timing was started, and the blood was collected. The total bleeding time and the bleeding volume within 30 min after the tails were clipped were recorded. Bleeding volume (mL) = (the weight of a centrifuge tube after blood collection (g) - the weight of the centrifuge tube before blood collection (g))/1.0. The experimental groups were compared through T-test testing and analysis software was Graphpad Prism 8.0, where *p<0.05* was considered to be statistically significant. Detailed results are shown in Table 2.

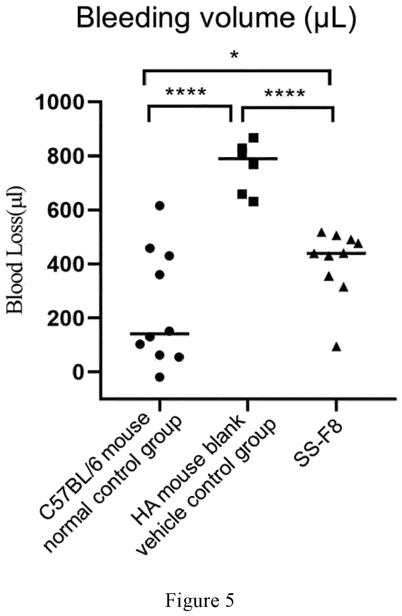

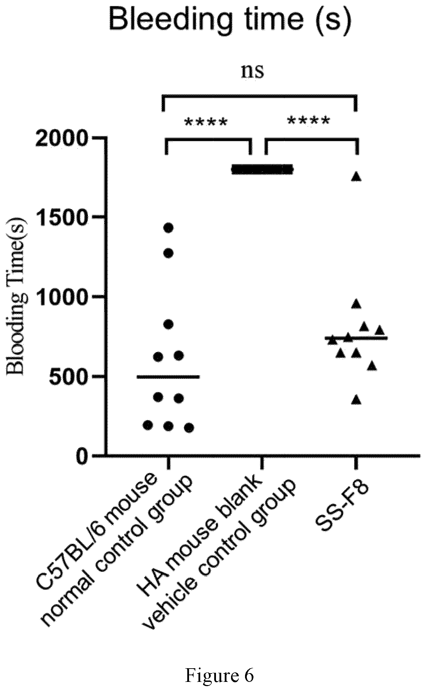

From the analysis of statistical results of the bleeding volume and the sum of bleeding time of each group of animals in FIGS. 5 and 6, the medians of the bleeding volume of the HA mouse blank vehicle control group and the C57BL/6 mouse normal control group were 790.00 µL and 141.15 µL, with an extremely significant difference (P < *0.0001*)*,* and the medians of the bleeding time were 1800 s and 497 s, with an extremely significant difference (P < *0.0001*)*,* which indicates that HA mice have an obvious bleeding characteristic compared with normal mice as for the tail-clipping model. After the administration of SS-F8 at the dosage of 112 IU/kg, the medians of the bleeding volume of the administration group and the HA mouse blank vehicle control group were 438.9 µL and 790.00 µL (*p* < *0.0001*)*,* and the medians of the bleeding time of the administration group and the HA mouse blank vehicle control group were 739 s and 1800 s (p < *0.0001*)*,* which indicates a significant coagulant effect and indicates that SS-F8 can be used as an effective coagulant for the acute hemorrhage in hemophilia A, i.e., coagulation factor VIII deficiency.

**Table 2 Data statistics of the bleeding volume and bleeding time of each group**

| Group | C57BL/6 Mouse Normal Control Group | HA Mouse Blank Vehicle Control Group | SS-F8 Administration Group |
|---|---|---|---|
| Bleeding volume (µL) | 141.15 | 790.00 | 438.90 |
| Bleeding time (s) | 497 | 1800 | 739 |

Though the preferred examples of the present disclosure are illustrated and described, it should be understood that those skilled in the art may make various changes in accordance with the teachings herein, and these changes do not violate the scope of the present disclosure.

All the publications mentioned in the present disclosure are incorporated herein by reference as if each publication is separately incorporated herein by reference. In addition, it should be understood that those skilled in the art, who have read the preceding content of the present disclosure, can make various changes or modifications on the present disclosure, and these equivalent forms fall within the scope of the appended claims.

## Claims

1. A fusion protein of human coagulation factor VIII, comprising sequentially from the N-terminus to the C-terminus a mutant single-chain human coagulation factor VIII with a partially deleted B domain, a flexible peptide linker, at least one carboxyl-terminal peptide rigid unit of human chorionic gonadotropin β-subunit, and a half-life extending moiety; wherein the single-chain human coagulation factor VIII has an amino acid sequence as shown in SEQ ID NO: 2, and the half-life extending moiety is selected from an immunoglobulin Fc fragment, albumin, transferrin, or PEG, preferably a human IgG Fc variant.

2. The fusion protein according to claim 1, wherein the fusion protein is glycosylated, preferably glycosylated by being expressed in mammal cells, such as Chinese hamster ovary cells.

3. The fusion protein according to claim 1, wherein the mutant single-chain human coagulation factor VIII with a partially deleted B domain comprises an amino acid sequence as shown in SEQ ID NO: 2, or the single-chain human coagulation factor VIII has an amino acid sequence that shares at least 90% identity to the amino acid sequence as shown in SEQ ID NO: 2.

4. The fusion protein according to claim 1, wherein the flexible peptide linker contains two or more amino acids selected from G, S, A, and T residues;
preferably, the flexible peptide linker has an amino acid sequence general formula formed by combining cyclic unit (GS)a(GGS)b(GGGS)c(GGGGS)d, wherein a, b, c, and d are integers greater than or equal to 0 and a+b+c+d ≥ 1;
more preferably, the flexible peptide linker is preferably selected from the following group:
(i) GSGGGSGGGGSGGGGS;
(ii) GSGGGGSGGGGSGGGGSGGGGSGGGGS;
(iii) GGGGSGGGGSGGGGSGGGGS;
(iv) GSGGGGSGGGGSGGGGSGGGGSGGGGSGGGGSGGGGS;
(v) GGGSGGGSGGGSGGGSGGGS.

5. The fusion protein according to claim 1, wherein the carboxyl-terminal peptide rigid unit of human chorionic gonadotropin β-subunit comprises an amino acid sequence as shown in SEQ ID NO: 3 or a truncated sequence thereof, wherein the truncated sequence comprises at least two glycosylation sites; preferably, the carboxyl-terminal peptide rigid unit of human chorionic gonadotropin β-subunit comprises a following amino acid sequence:
(i) PRFQDSSSSKAPPPSLPSPSRLPGPSDTPILPQ;
(ii) SSSSKAPPPSLPSPSRLPGPSDTPILPQ;
(iii) SSSSKAPPPS;
(iv) SRLPGPSDTPILPQ.

6. The fusion protein according to claim 1, wherein the carboxyl-terminal peptide rigid unit of human chorionic gonadotropin β-subunit shares at least 70%, 80%, 90%, or 95% identity to an amino acid sequence of a carboxyl-terminal peptide rigid unit in the fusion protein according to claim 5.

7. The fusion protein according to claim 1, wherein the fusion protein comprises 1, 2, 3, 4, or 5 carboxyl-terminal peptide rigid units of human chorionic gonadotropin β-subunit.

8. The fusion protein according to claim 1, wherein the human IgG Fc variant has a reduced ADCC effect and/or CDC effect and/or enhanced binding affinity with an FcRn receptor; preferably, the Fc variant is selected from:
(i) human IgG1 hinge region, CH2 region, and CH3 region containing Leu234Val, Leu235Ala, and Pro331 Ser mutations;
(ii) human IgG2 hinge region, CH2 region, and CH3 region containing Pro331 Ser mutation;
(iii) human IgG2 hinge region, CH2 region, and CH3 region containing Thr250Gln and Met428Leu mutations;
(iv) human IgG2 hinge region, CH2 region, and CH3 region containing Pro331Ser, Thr250Gln, and Met428Leu mutations;
(v) human IgG4 hinge region, CH2 region, and CH3 region containing Ser228Pro and Leu235Ala mutations.

9. The fusion protein according to claim 1, wherein the fusion protein has an amino acid sequence as shown in SEQ ID NO: 9.

10. The fusion protein according to any one of claims 1 to 9, wherein the fusion protein has activity of greater than 6000 IU/mg.

11. DNA for encoding the fusion protein according to any one of claims 1 to 10, wherein preferably, the DNA has a sequence as shown in SEQ ID NO: 10.

12. A vector, comprising the DNA according to claim 11.

13. A host cell, which comprises or has been transfected with the vector according to claim 12.

14. A pharmaceutical composition, comprising a pharmaceutically acceptable carrier, excipient, or diluent and an effective dose of the fusion protein according to any one of claims 1 to 10.

15. A method for preparing the fusion protein according to any one of claims 1 to 10, comprising:
(a) introducing the DNA sequence for encoding a fusion protein according to claim 11 into a CHO cell to produce a CHO-derived cell line;
(b) screening in step (a) a high-yielding cell strain that expresses more than 1 IU/10⁶ (million) cells every 24 hours in a growth medium thereof;
(c) culturing the cell strain screened in step (b) to express the fusion protein;
(d) harvesting a fermentation broth obtained in step (c) and separating and purifying the fusion protein.

16. The method according to claim 15, wherein a purification process of the fusion protein in step (d) comprises affinity chromatography, hydrophobic chromatography, anion exchange chromatography, and molecular sieve chromatography.

17. Use of the fusion protein according to any one of claims 1 to 10 for preparing a medicament for prevention or treatment of a hemorrhagic disease, such as a hemorrhagic disease in a patient with a congenital or acquired FVIII deficiency, or spontaneous or surgical bleeding in a patient with hemophilia A.


SEQUENCE LISTING

<110>  AMPSOURCE BIOPHARMA SHANGHAI INC.
       PHARMAB, INC.

<120>  FUSION PROTEIN OF MUTATED SINGLE-CHAIN HUMAN COAGULATION FACTOR
       VIII, PREPARATION METHOD THEREFOR, AND USE THEREOF

<130>  2018

<160>  20

<170>  PatentIn version 3.5

<210>  1
<211>  2332
<212>  PRT
<213>  Homo sapiens

<400>  1

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr

            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His
            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Ser Thr Glu Ser Gly Arg Leu Phe Lys Gly Lys
                965                 970                 975

Arg Ala His Gly Pro Ala Leu Leu Thr Lys Asp Asn Ala Leu Phe Lys
            980                 985                 990

Val Ser Ile Ser Leu Leu Lys Thr Asn Lys Thr Ser Asn Asn Ser Ala
        995                 1000                1005

Thr Asn Arg Lys Thr His Ile Asp Gly Pro Ser Leu Leu Ile Glu
    1010                1015                1020

Asn Ser Pro Ser Val Trp Gln Asn Ile Leu Glu Ser Asp Thr Glu
    1025                1030                1035

Phe Lys Lys Val Thr Pro Leu Ile His Asp Arg Met Leu Met Asp
    1040                1045                1050

Lys Asn Ala Thr Ala Leu Arg Leu Asn His Met Ser Asn Lys Thr
    1055                1060                1065

Thr Ser Ser Lys Asn Met Glu Met Val Gln Gln Lys Lys Glu Gly
    1070                1075                1080

Pro Ile Pro Pro Asp Ala Gln Asn Pro Asp Met Ser Phe Phe Lys
    1085                1090                1095

Met Leu Phe Leu Pro Glu Ser Ala Arg Trp Ile Gln Arg Thr His
    1100                1105                1110

Gly Lys Asn Ser Leu Asn Ser Gly Gln Gly Pro Ser Pro Lys Gln
    1115                1120                1125

Leu Val Ser Leu Gly Pro Glu Lys Ser Val Glu Gly Gln Asn Phe
    1130                1135                1140

Leu Ser Glu Lys Asn Lys Val Val Val Gly Lys Gly Glu Phe Thr
    1145                1150                1155

Lys Asp Val Gly Leu Lys Glu Met Val Phe Pro Ser Ser Arg Asn
    1160                1165                1170

Leu Phe Leu Thr Asn Leu Asp Asn Leu His Glu Asn Asn Thr His
    1175                1180                1185

Asn Gln Glu Lys Lys Ile Gln Glu Glu Ile Glu Lys Lys Glu Thr
    1190                1195                1200

Leu Ile Gln Glu Asn Val Val Leu Pro Gln Ile His Thr Val Thr
    1205                1210                1215

Gly Thr Lys Asn Phe Met Lys Asn Leu Phe Leu Leu Ser Thr Arg
    1220                1225                1230

Gln Asn Val Glu Gly Ser Tyr Asp Gly Ala Tyr Ala Pro Val Leu
    1235                1240                1245

Gln Asp Phe Arg Ser Leu Asn Asp Ser Thr Asn Arg Thr Lys Lys
    1250                1255                1260

His Thr Ala His Phe Ser Lys Lys Gly Glu Glu Glu Asn Leu Glu
    1265                1270                1275

Gly Leu Gly Asn Gln Thr Lys Gln Ile Val Glu Lys Tyr Ala Cys
    1280                1285                1290

Thr Thr Arg Ile Ser Pro Asn Thr Ser Gln Gln Asn Phe Val Thr
    1295                1300                1305

Gln Arg Ser Lys Arg Ala Leu Lys Gln Phe Arg Leu Pro Leu Glu
    1310                1315                1320

Glu Thr Glu Leu Glu Lys Arg Ile Ile Val Asp Asp Thr Ser Thr
    1325                1330                1335

Gln Trp Ser Lys Asn Met Lys His Leu Thr Pro Ser Thr Leu Thr
    1340                1345                1350

Gln Ile Asp Tyr Asn Glu Lys Glu Lys Gly Ala Ile Thr Gln Ser
    1355                1360                1365

Pro Leu Ser Asp Cys Leu Thr Arg Ser His Ser Ile Pro Gln Ala
    1370                1375                1380

Asn Arg Ser Pro Leu Pro Ile Ala Lys Val Ser Ser Phe Pro Ser
    1385                1390                1395

Ile Arg Pro Ile Tyr Leu Thr Arg Val Leu Phe Gln Asp Asn Ser

1400                1405                1410

Ser His Leu Pro Ala Ala Ser Tyr Arg Lys Lys Asp Ser Gly Val
    1415                1420                1425

Gln Glu Ser Ser His Phe Leu Gln Gly Ala Lys Lys Asn Asn Leu
    1430                1435                1440

Ser Leu Ala Ile Leu Thr Leu Glu Met Thr Gly Asp Gln Arg Glu
    1445                1450                1455

Val Gly Ser Leu Gly Thr Ser Ala Thr Asn Ser Val Thr Tyr Lys
    1460                1465                1470

Lys Val Glu Asn Thr Val Leu Pro Lys Pro Asp Leu Pro Lys Thr
    1475                1480                1485

Ser Gly Lys Val Glu Leu Leu Pro Lys Val His Ile Tyr Gln Lys
    1490                1495                1500

Asp Leu Phe Pro Thr Glu Thr Ser Asn Gly Ser Pro Gly His Leu
    1505                1510                1515

Asp Leu Val Glu Gly Ser Leu Leu Gln Gly Thr Glu Gly Ala Ile
    1520                1525                1530

Lys Trp Asn Glu Ala Asn Arg Pro Gly Lys Val Pro Phe Leu Arg
    1535                1540                1545

Val Ala Thr Glu Ser Ser Ala Lys Thr Pro Ser Lys Leu Leu Asp
    1550                1555                1560

Pro Leu Ala Trp Asp Asn His Tyr Gly Thr Gln Ile Pro Lys Glu
    1565                1570                1575

Glu Trp Lys Ser Gln Glu Lys Ser Pro Glu Lys Thr Ala Phe Lys
    1580                1585                1590

Lys Lys Asp Thr Ile Leu Ser Leu Asn Ala Cys Glu Ser Asn His
    1595                1600                1605

Ala Ile Ala Ala Ile Asn Glu Gly Gln Asn Lys Pro Glu Ile Glu
    1610                1615                1620

Val Thr Trp Ala Lys Gln Gly Arg Thr Glu Arg Leu Cys Ser Gln
    1625                1630                1635

Asn Pro Pro Val Leu Lys Arg His Gln Arg Glu Ile Thr Arg Thr
    1640                1645                1650

Thr Leu Gln Ser Asp Gln Glu Glu Ile Asp Tyr Asp Asp Thr Ile
    1655                1660                1665

Ser Val Glu Met Lys Lys Glu Asp Phe Asp Ile Tyr Asp Glu Asp
    1670                1675                1680

Glu Asn Gln Ser Pro Arg Ser Phe Gln Lys Lys Thr Arg His Tyr
    1685                1690                1695

Phe Ile Ala Ala Val Glu Arg Leu Trp Asp Tyr Gly Met Ser Ser
    1700                1705                1710

Ser Pro His Val Leu Arg Asn Arg Ala Gln Ser Gly Ser Val Pro
    1715                1720                1725

Gln Phe Lys Lys Val Val Phe Gln Glu Phe Thr Asp Gly Ser Phe
    1730                1735                1740

Thr Gln Pro Leu Tyr Arg Gly Glu Leu Asn Glu His Leu Gly Leu
    1745                1750                1755

Leu Gly Pro Tyr Ile Arg Ala Glu Val Glu Asp Asn Ile Met Val
    1760                1765                1770

Thr Phe Arg Asn Gln Ala Ser Arg Pro Tyr Ser Phe Tyr Ser Ser
    1775                1780                1785

Leu Ile Ser Tyr Glu Glu Asp Gln Arg Gln Gly Ala Glu Pro Arg
    1790                1795                1800

Lys Asn Phe Val Lys Pro Asn Glu Thr Lys Thr Tyr Phe Trp Lys
    1805                1810                1815

Val Gln His His Met Ala Pro Thr Lys Asp Glu Phe Asp Cys Lys
    1820                1825                1830

Ala Trp Ala Tyr Phe Ser Asp Val Asp Leu Glu Lys Asp Val His
    1835                1840                1845

Ser Gly Leu Ile Gly Pro Leu Leu Val Cys His Thr Asn Thr Leu
    1850                1855                1860

Asn Pro Ala His Gly Arg Gln Val Thr Val Gln Glu Phe Ala Leu
    1865                1870                1875

Phe Phe Thr Ile Phe Asp Glu Thr Lys Ser Trp Tyr Phe Thr Glu
    1880                1885                1890

Asn Met Glu Arg Asn Cys Arg Ala Pro Cys Asn Ile Gln Met Glu
    1895                1900                1905

Asp Pro Thr Phe Lys Glu Asn Tyr Arg Phe His Ala Ile Asn Gly
    1910                1915                1920

Tyr Ile Met Asp Thr Leu Pro Gly Leu Val Met Ala Gln Asp Gln
    1925                1930                1935

Arg Ile Arg Trp Tyr Leu Leu Ser Met Gly Ser Asn Glu Asn Ile
    1940                1945                1950

His Ser Ile His Phe Ser Gly His Val Phe Thr Val Arg Lys Lys
    1955                1960                1965

Glu Glu Tyr Lys Met Ala Leu Tyr Asn Leu Tyr Pro Gly Val Phe
    1970                1975                1980

Glu Thr Val Glu Met Leu Pro Ser Lys Ala Gly Ile Trp Arg Val
    1985                1990                1995

Glu Cys Leu Ile Gly Glu His Leu His Ala Gly Met Ser Thr Leu
    2000                2005                2010

Phe Leu Val Tyr Ser Asn Lys Cys Gln Thr Pro Leu Gly Met Ala
    2015                2020                2025

Ser Gly His Ile Arg Asp Phe Gln Ile Thr Ala Ser Gly Gln Tyr
    2030                2035                2040

Gly Gln Trp Ala Pro Lys Leu Ala Arg Leu His Tyr Ser Gly Ser
    2045                2050                2055

Ile Asn Ala Trp Ser Thr Lys Glu Pro Phe Ser Trp Ile Lys Val
    2060                2065                2070

Asp Leu Leu Ala Pro Met Ile Ile His Gly Ile Lys Thr Gln Gly
    2075                2080                2085

Ala Arg Gln Lys Phe Ser Ser Leu Tyr Ile Ser Gln Phe Ile Ile
    2090                2095                2100

Met Tyr Ser Leu Asp Gly Lys Lys Trp Gln Thr Tyr Arg Gly Asn
    2105                2110                2115

Ser Thr Gly Thr Leu Met Val Phe Phe Gly Asn Val Asp Ser Ser
    2120                2125                2130

Gly Ile Lys His Asn Ile Phe Asn Pro Pro Ile Ile Ala Arg Tyr
    2135                2140                2145

Ile Arg Leu His Pro Thr His Tyr Ser Ile Arg Ser Thr Leu Arg
    2150                2155                2160

Met Glu Leu Met Gly Cys Asp Leu Asn Ser Cys Ser Met Pro Leu
    2165                2170                2175

Gly Met Glu Ser Lys Ala Ile Ser Asp Ala Gln Ile Thr Ala Ser
    2180                2185                2190

Ser Tyr Phe Thr Asn Met Phe Ala Thr Trp Ser Pro Ser Lys Ala
    2195                2200                2205

Arg Leu His Leu Gln Gly Arg Ser Asn Ala Trp Arg Pro Gln Val
    2210                2215                2220

Asn Asn Pro Lys Glu Trp Leu Gln Val Asp Phe Gln Lys Thr Met
    2225                2230                2235

Lys Val Thr Gly Val Thr Thr Gln Gly Val Lys Ser Leu Leu Thr
    2240                2245                2250

Ser Met Tyr Val Lys Glu Phe Leu Ile Ser Ser Ser Gln Asp Gly
    2255                2260                2265

His Gln Trp Thr Leu Phe Phe Gln Asn Gly Lys Val Lys Val Phe
    2270                2275                2280

Gln Gly Asn Gln Asp Ser Phe Thr Pro Val Val Asn Ser Leu Asp
    2285                2290                2295

Pro Pro Leu Leu Thr Arg Tyr Leu Arg Ile His Pro Gln Ser Trp
    2300                2305                2310

Val His Gln Ile Ala Leu Arg Met Glu Val Leu Gly Cys Glu Ala
    2315                2320                2325

Gln Asp Leu Tyr
    2330

<210>  2

<211>  1445
<212>  PRT
<213>  Artificial sequence

<220>
<223>  scFVIII amino acid sequence

<400>  2

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile

465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Arg Thr Thr Leu
        755                 760                 765

Gln Ser Asp Gln Glu Glu Ile Asp Tyr Asp Asp Thr Ile Ser Val Glu
    770                 775                 780

Met Lys Lys Glu Asp Phe Asp Ile Tyr Asp Glu Asp Glu Asn Gln Ser
785                 790                 795                 800

Pro Arg Ser Phe Gln Lys Lys Thr Arg His Tyr Phe Ile Ala Ala Val
                805                 810                 815

Glu Arg Leu Trp Asp Tyr Gly Met Ser Ser Ser Pro His Val Leu Arg
            820                 825                 830

Asn Arg Ala Gln Ser Gly Ser Val Pro Gln Phe Lys Lys Val Val Phe
        835                 840                 845

Gln Glu Phe Thr Asp Gly Ser Phe Thr Gln Pro Leu Tyr Arg Gly Glu
    850                 855                 860

Leu Asn Glu His Leu Gly Leu Leu Gly Pro Tyr Ile Arg Ala Glu Val
865                 870                 875                 880

Glu Asp Asn Ile Met Val Thr Phe Arg Asn Gln Ala Ser Arg Pro Tyr
                885                 890                 895

Ser Phe Tyr Ser Ser Leu Ile Ser Tyr Glu Glu Asp Gln Arg Gln Gly
            900                 905                 910

Ala Glu Pro Arg Lys Asn Phe Val Lys Pro Asn Glu Thr Lys Thr Tyr
        915                 920                 925

Phe Trp Lys Val Gln His His Met Ala Pro Thr Lys Asp Glu Phe Asp
    930                 935                 940

Cys Lys Ala Trp Ala Tyr Phe Ser Asp Val Asp Leu Glu Lys Asp Val
945                 950                 955                 960

His Ser Gly Leu Ile Gly Pro Leu Leu Val Cys His Thr Asn Thr Leu
                965                 970                 975

Asn Pro Ala His Gly Arg Gln Val Thr Val Gln Glu Phe Ala Leu Phe
            980                 985                 990

Phe Thr Ile Phe Asp Glu Thr Lys  Ser Trp Tyr Phe Thr  Glu Asn Met
        995                 1000                 1005

Glu Arg  Asn Cys Arg Ala Pro  Cys Asn Ile Gln Met  Glu Asp Pro
    1010                 1015                 1020

Thr Phe  Lys Glu Asn Tyr Arg  Phe His Ala Ile Asn  Gly Tyr Ile
    1025                 1030                 1035

Met Asp  Thr Leu Pro Gly Leu  Val Met Ala Gln Asp  Gln Arg Ile
    1040                 1045                 1050

Arg Trp  Tyr Leu Leu Ser Met  Gly Ser Asn Glu Asn  Ile His Ser
    1055                 1060                 1065

Ile His  Phe Ser Gly His Val  Phe Thr Val Arg Lys  Lys Glu Glu
    1070                 1075                 1080

Tyr Lys  Met Ala Leu Tyr Asn  Leu Tyr Pro Gly Val  Phe Glu Thr
    1085                 1090                 1095

Val Glu  Met Leu Pro Ser Lys  Ala Gly Ile Trp Arg  Val Glu Cys
    1100                 1105                 1110

Leu Ile  Gly Glu His Leu His  Ala Gly Met Ser Thr  Leu Phe Leu
    1115                 1120                 1125

Val Tyr  Ser Asn Lys Cys Gln  Thr Pro Leu Gly Met  Ala Ser Gly
    1130                 1135                 1140

His Ile  Arg Asp Phe Gln Ile  Thr Ala Ser Gly Gln  Tyr Gly Gln
    1145                 1150                 1155

Trp Ala  Pro Lys Leu Ala Arg  Leu His Tyr Ser Gly  Ser Ile Asn
    1160                 1165                 1170

Ala Trp  Ser Thr Lys Glu Pro  Phe Ser Trp Ile Lys  Val Asp Leu
    1175                 1180                 1185

Leu Ala  Pro Met Ile Ile His  Gly Ile Lys Thr Gln  Gly Ala Arg
    1190                 1195                 1200

Gln Lys  Phe Ser Ser Leu Tyr  Ile Ser Gln Phe Ile  Ile Met Tyr
    1205                 1210                 1215

Ser Leu Asp Gly Lys Lys Trp Gln Thr Tyr Arg Gly Asn Ser Thr
    1220                1225                1230

Gly Thr Leu Met Val Phe Phe Gly Asn Val Asp Ser Ser Gly Ile
    1235                1240                1245

Lys His Asn Ile Phe Asn Pro Pro Ile Ile Ala Arg Tyr Ile Arg
    1250                1255                1260

Leu His Pro Thr His Tyr Ser Ile Arg Ser Thr Leu Arg Met Glu
    1265                1270                1275

Leu Met Gly Cys Asp Leu Asn Ser Cys Ser Met Pro Leu Gly Met
    1280                1285                1290

Glu Ser Lys Ala Ile Ser Asp Ala Gln Ile Thr Ala Ser Ser Tyr
    1295                1300                1305

Phe Thr Asn Met Phe Ala Thr Trp Ser Pro Ser Lys Ala Arg Leu
    1310                1315                1320

His Leu Gln Gly Arg Ser Asn Ala Trp Arg Pro Gln Val Asn Asn
    1325                1330                1335

Pro Lys Glu Trp Leu Gln Val Asp Phe Gln Lys Thr Met Lys Val
    1340                1345                1350

Thr Gly Val Thr Thr Gln Gly Val Lys Ser Leu Leu Thr Ser Met
    1355                1360                1365

Tyr Val Lys Glu Phe Leu Ile Ser Ser Ser Gln Asp Gly His Gln
    1370                1375                1380

Trp Thr Leu Phe Phe Gln Asn Gly Lys Val Lys Val Phe Gln Gly
    1385                1390                1395

Asn Gln Asp Ser Phe Thr Pro Val Val Asn Ser Leu Asp Pro Pro
    1400                1405                1410

Leu Leu Thr Arg Tyr Leu Arg Ile His Pro Gln Ser Trp Val His
    1415                1420                1425

Gln Ile Ala Leu Arg Met Glu Val Leu Gly Cys Glu Ala Gln Asp
    1430                1435                1440

Leu Tyr

    1445

<210>  3
<211>  33
<212>  PRT
<213>  Homo sapiens

<400>  3

Pro Arg Phe Gln Asp Ser Ser Ser Ser Lys Ala Pro Pro Pro Ser Leu
1               5                   10                  15

Pro Ser Pro Ser Arg Leu Pro Gly Pro Ser Asp Thr Pro Ile Leu Pro
            20                  25                  30

Gln

<210>  4
<211>  227
<212>  PRT
<213>  Artificial sequence

<220>
<223>  vFcgamma1 amino acid sequence

<400>  4

Asp Lys Thr His Thr Cys Pro Pro Cys Pro Ala Pro Glu Val Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Ser Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  5
<211>  223
<212>  PRT
<213>  Artificial sequence

<220>
<223>  vFcgamma2-1 amino acid sequence

<400>  5

Val Glu Cys Pro Pro Cys Pro Ala Pro Pro Val Ala Gly Pro Ser Val
1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Gln Phe Asn Trp Tyr Val Asp Gly Val Glu Val His Asn Ala Lys
    50                  55                  60

Thr Lys Pro Arg Glu Glu Gln Phe Asn Ser Thr Phe Arg Val Val Ser
65                  70                  75                  80

Val Leu Thr Val Val His Gln Asp Trp Leu Asn Gly Lys Glu Tyr Lys
                85                  90                  95

Cys Lys Val Ser Asn Lys Gly Leu Pro Ala Ser Ile Glu Lys Thr Ile
            100                 105                 110

Ser Lys Thr Lys Gly Gln Pro Arg Glu Pro Gln Val Tyr Thr Leu Pro
        115                 120                 125

Pro Ser Arg Glu Glu Met Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220

<210> 6
<211> 223
<212> PRT
<213> Artificial sequence

<220>
<223> vFcgamma2-2 amino acid sequence

<400> 6

Val Glu Cys Pro Pro Cys Pro Ala Pro Pro Val Ala Gly Pro Ser Val
1               5                   10                  15

Phe Leu Phe Pro Pro Lys Pro Lys Asp Gln Leu Met Ile Ser Arg Thr
            20                  25                  30

Pro Glu Val Thr Cys Val Val Val Asp Val Ser His Glu Asp Pro Glu
        35                  40                  45

Val Gln Phe Asn Trp Tyr Val Asp Gly Val Glu Val His Asn Ala Lys
    50                  55                  60

Thr Lys Pro Arg Glu Glu Gln Phe Asn Ser Thr Phe Arg Val Val Ser
65                  70                  75                  80

Val Leu Thr Val Val His Gln Asp Trp Leu Asn Gly Lys Glu Tyr Lys

85                  90                  95

Cys Lys Val Ser Asn Lys Gly Leu Pro Ala Pro Ile Glu Lys Thr Ile
            100                 105                 110

Ser Lys Thr Lys Gly Gln Pro Arg Glu Pro Gln Val Tyr Thr Leu Pro
        115                 120                 125

Pro Ser Arg Glu Glu Met Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Leu His Glu Ala Leu
        195                 200                 205

His Asn His Tyr Thr Gln Lys Ser Leu Ser Leu Ser Pro Gly Lys
    210                 215                 220

<210>  7
<211>  223
<212>  PRT
<213>  Artificial sequence

<220>
<223>  vFcgamma2-3 amino acid sequence

<400>  7

Val Glu Cys Pro Pro Cys Pro Ala Pro Pro Val Ala Gly Pro Ser Val
1               5                   10                  15

Phe Leu Phe Pro Pro Lys Pro Lys Asp Gln Leu Met Ile Ser Arg Thr
            20                  25                  30

Pro Glu Val Thr Cys Val Val Val Asp Val Ser His Glu Asp Pro Glu
        35                  40                  45

Val Gln Phe Asn Trp Tyr Val Asp Gly Val Glu Val His Asn Ala Lys
    50                  55                  60

Thr Lys Pro Arg Glu Glu Gln Phe Asn Ser Thr Phe Arg Val Val Ser
65                  70                  75                  80

Val Leu Thr Val Val His Gln Asp Trp Leu Asn Gly Lys Glu Tyr Lys
                85                  90                  95

Cys Lys Val Ser Asn Lys Gly Leu Pro Ala Ser Ile Glu Lys Thr Ile
            100                 105                 110

Ser Lys Thr Lys Gly Gln Pro Arg Glu Pro Gln Val Tyr Thr Leu Pro
        115                 120                 125

Pro Ser Arg Glu Glu Met Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Leu His Glu Ala Leu
        195                 200                 205

His Asn His Tyr Thr Gln Lys Ser Leu Ser Leu Ser Pro Gly Lys
    210                 215                 220

<210>  8
<211>  229
<212>  PRT
<213>  Artificial sequence

<220>
<223>  vFcgamma4 amino acid sequence

<400>  8

Glu Ser Lys Tyr Gly Pro Pro Cys Pro Pro Cys Pro Ala Pro Glu Phe
1               5                   10                  15

Ala Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210>  9
<211>  1723
<212>  PRT
<213>  Artificial sequence

<220>
<223>  amino acid sequence of scFVIII fusion protein

<400>  9

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Arg Thr Thr Leu
        755                 760                 765

Gln Ser Asp Gln Glu Glu Ile Asp Tyr Asp Asp Thr Ile Ser Val Glu
    770                 775                 780

Met Lys Lys Glu Asp Phe Asp Ile Tyr Asp Glu Asp Glu Asn Gln Ser

785                 790                 795                 800

Pro Arg Ser Phe Gln Lys Lys Thr Arg His Tyr Phe Ile Ala Ala Val
                805                 810                 815

Glu Arg Leu Trp Asp Tyr Gly Met Ser Ser Ser Pro His Val Leu Arg
            820                 825                 830

Asn Arg Ala Gln Ser Gly Ser Val Pro Gln Phe Lys Lys Val Val Phe
        835                 840                 845

Gln Glu Phe Thr Asp Gly Ser Phe Thr Gln Pro Leu Tyr Arg Gly Glu
    850                 855                 860

Leu Asn Glu His Leu Gly Leu Leu Gly Pro Tyr Ile Arg Ala Glu Val
865                 870                 875                 880

Glu Asp Asn Ile Met Val Thr Phe Arg Asn Gln Ala Ser Arg Pro Tyr
                885                 890                 895

Ser Phe Tyr Ser Ser Leu Ile Ser Tyr Glu Glu Asp Gln Arg Gln Gly
            900                 905                 910

Ala Glu Pro Arg Lys Asn Phe Val Lys Pro Asn Glu Thr Lys Thr Tyr
        915                 920                 925

Phe Trp Lys Val Gln His His Met Ala Pro Thr Lys Asp Glu Phe Asp
    930                 935                 940

Cys Lys Ala Trp Ala Tyr Phe Ser Asp Val Asp Leu Glu Lys Asp Val
945                 950                 955                 960

His Ser Gly Leu Ile Gly Pro Leu Leu Val Cys His Thr Asn Thr Leu
                965                 970                 975

Asn Pro Ala His Gly Arg Gln Val Thr Val Gln Glu Phe Ala Leu Phe
            980                 985                 990

Phe Thr Ile Phe Asp Glu Thr Lys  Ser Trp Tyr Phe Thr  Glu Asn Met
        995                 1000                1005

Glu Arg  Asn Cys Arg Ala Pro  Cys Asn Ile Gln Met  Glu Asp Pro
    1010                1015                1020

Thr Phe  Lys Glu Asn Tyr Arg  Phe His Ala Ile Asn  Gly Tyr Ile
    1025                1030                1035

Met Asp Thr Leu Pro Gly Leu Val Met Ala Gln Asp Gln Arg Ile
    1040                1045                1050

Arg Trp Tyr Leu Leu Ser Met Gly Ser Asn Glu Asn Ile His Ser
    1055                1060                1065

Ile His Phe Ser Gly His Val Phe Thr Val Arg Lys Lys Glu Glu
    1070                1075                1080

Tyr Lys Met Ala Leu Tyr Asn Leu Tyr Pro Gly Val Phe Glu Thr
    1085                1090                1095

Val Glu Met Leu Pro Ser Lys Ala Gly Ile Trp Arg Val Glu Cys
    1100                1105                1110

Leu Ile Gly Glu His Leu His Ala Gly Met Ser Thr Leu Phe Leu
    1115                1120                1125

Val Tyr Ser Asn Lys Cys Gln Thr Pro Leu Gly Met Ala Ser Gly
    1130                1135                1140

His Ile Arg Asp Phe Gln Ile Thr Ala Ser Gly Gln Tyr Gly Gln
    1145                1150                1155

Trp Ala Pro Lys Leu Ala Arg Leu His Tyr Ser Gly Ser Ile Asn
    1160                1165                1170

Ala Trp Ser Thr Lys Glu Pro Phe Ser Trp Ile Lys Val Asp Leu
    1175                1180                1185

Leu Ala Pro Met Ile Ile His Gly Ile Lys Thr Gln Gly Ala Arg
    1190                1195                1200

Gln Lys Phe Ser Ser Leu Tyr Ile Ser Gln Phe Ile Ile Met Tyr
    1205                1210                1215

Ser Leu Asp Gly Lys Lys Trp Gln Thr Tyr Arg Gly Asn Ser Thr
    1220                1225                1230

Gly Thr Leu Met Val Phe Phe Gly Asn Val Asp Ser Ser Gly Ile
    1235                1240                1245

Lys His Asn Ile Phe Asn Pro Pro Ile Ile Ala Arg Tyr Ile Arg
    1250                1255                1260

Leu His Pro Thr His Tyr Ser Ile Arg Ser Thr Leu Arg Met Glu
    1265                1270                1275

Leu Met Gly Cys Asp Leu Asn Ser Cys Ser Met Pro Leu Gly Met
    1280                1285                1290

Glu Ser Lys Ala Ile Ser Asp Ala Gln Ile Thr Ala Ser Ser Tyr
    1295                1300                1305

Phe Thr Asn Met Phe Ala Thr Trp Ser Pro Ser Lys Ala Arg Leu
    1310                1315                1320

His Leu Gln Gly Arg Ser Asn Ala Trp Arg Pro Gln Val Asn Asn
    1325                1330                1335

Pro Lys Glu Trp Leu Gln Val Asp Phe Gln Lys Thr Met Lys Val
    1340                1345                1350

Thr Gly Val Thr Thr Gln Gly Val Lys Ser Leu Leu Thr Ser Met
    1355                1360                1365

Tyr Val Lys Glu Phe Leu Ile Ser Ser Ser Gln Asp Gly His Gln
    1370                1375                1380

Trp Thr Leu Phe Phe Gln Asn Gly Lys Val Lys Val Phe Gln Gly
    1385                1390                1395

Asn Gln Asp Ser Phe Thr Pro Val Val Asn Ser Leu Asp Pro Pro
    1400                1405                1410

Leu Leu Thr Arg Tyr Leu Arg Ile His Pro Gln Ser Trp Val His
    1415                1420                1425

Gln Ile Ala Leu Arg Met Glu Val Leu Gly Cys Glu Ala Gln Asp
    1430                1435                1440

Leu Tyr Gly Ser Gly Gly Gly Gly Ser Gly Gly Gly Gly Ser Gly
    1445                1450                1455

Gly Gly Gly Ser Gly Gly Gly Gly Ser Gly Gly Gly Gly Ser Ser
    1460                1465                1470

Ser Ser Ser Lys Ala Pro Pro Pro Ser Leu Pro Ser Pro Ser Arg
    1475                1480                1485

Leu Pro Gly Pro Ser Asp Thr Pro Ile Leu Pro Gln Val Glu Cys
    1490                1495                1500

Pro Pro Cys Pro Ala Pro Pro Val Ala Gly Pro Ser Val Phe Leu
    1505                1510                1515

Phe Pro Pro Lys Pro Lys Asp Gln Leu Met Ile Ser Arg Thr Pro
    1520                1525                1530

Glu Val Thr Cys Val Val Val Asp Val Ser His Glu Asp Pro Glu
    1535                1540                1545

Val Gln Phe Asn Trp Tyr Val Asp Gly Val Glu Val His Asn Ala
    1550                1555                1560

Lys Thr Lys Pro Arg Glu Glu Gln Phe Asn Ser Thr Phe Arg Val
    1565                1570                1575

Val Ser Val Leu Thr Val Val His Gln Asp Trp Leu Asn Gly Lys
    1580                1585                1590

Glu Tyr Lys Cys Lys Val Ser Asn Lys Gly Leu Pro Ala Ser Ile
    1595                1600                1605

Glu Lys Thr Ile Ser Lys Thr Lys Gly Gln Pro Arg Glu Pro Gln
    1610                1615                1620

Val Tyr Thr Leu Pro Pro Ser Arg Glu Glu Met Thr Lys Asn Gln
    1625                1630                1635

Val Ser Leu Thr Cys Leu Val Lys Gly Phe Tyr Pro Ser Asp Ile
    1640                1645                1650

Ala Val Glu Trp Glu Ser Asn Gly Gln Pro Glu Asn Asn Tyr Lys
    1655                1660                1665

Thr Thr Pro Pro Met Leu Asp Ser Asp Gly Ser Phe Phe Leu Tyr
    1670                1675                1680

Ser Lys Leu Thr Val Asp Lys Ser Arg Trp Gln Gln Gly Asn Val
    1685                1690                1695

Phe Ser Cys Ser Val Leu His Glu Ala Leu His Asn His Tyr Thr
    1700                1705                1710

Gln Lys Ser Leu Ser Leu Ser Pro Gly Lys
    1715                1720

<210>  10
<211>  5169
<212>  DNA
<213>  Artificial sequence

<220>
<223>  nucleotide sequence of scFVIII fusion protein

<400>  10
gccaccagga ggtactacct cggagccgtc gagctcagct gggattatat gcagagcgac      60

ctcggagaac tccctgtcga tgccaggttt cccccccggg tgcccaagtc cttccccttt     120

aacacctccg tcgtctacaa gaagaccctg ttcgtcgagt tcacagacca tctgttcaat     180

attgctaaac ccaggccccc ctggatggga ctgctgggcc ccacaatcca agccgaggtg     240

tatgataccg tggtgatcac cctcaagaat atggcttccc atcccgtctc cctgcacgcc     300

gtcggcgtgt cctactggaa ggcctccgaa ggcgccgagt acgatgatca gacaagccag     360

cgggagaagg aggatgataa ggtctttccc ggaggttctc acacctatgt ctggcaggtg     420

ctgaaagaga acggccccat ggctagcgac cccctgtgcc tcacctattc ctacctcagc     480

catgtggacc tggtgaagga tctgaactcc ggcctgattg gcgccctcct ggtctgcagg     540

gagggaagcc tggccaaaga aaagacccag accctgcata aattcatcct cctgtttgcc     600

gtcttcgatg agggcaagtc ctggcactcc gaaacaaaga actccctgat gcaggacagg     660

gacgctgcct ccgccagggc ctggcccaag atgcacaccg tcaacggata cgtgaatagg     720

tccctccccg gcctgattgg atgccatcgg aagtccgtgt attggcacgt gatcggcatg     780

ggaaccacac ccgaagtgca ctccatcttc ctggaaggac atacatttct cgtccggaac     840

caccggcagg cttccctgga aatctcccct atcaccttcc tgaccgctca gacactgctg     900

atggatctgg gccagttcct gctgttctgc cacatttcct cccaccagca cgacggcatg     960

gaggcttatg tcaaggtgga tagctgcccc gaggaacccc agctgcggat gaaaaacaat    1020

gaggaagccg aggactacga cgatgacctc accgatagcg agatggacgt ggtcaggttc    1080

gacgacgata actccccttc cttcatccag atccggagcg tggccaagaa gcaccccaaa    1140

acctgggtgc actacatcgc cgctgaggag gaagactggg actatgctcc tctcgtgctg    1200

gcccccgatg acaggtccta caaaagccag tatctgaaca atggacccca gcggattgga    1260

aggaagtata agaaagtccg gtttatggct tacaccgacg agacctttaa aacccgggaa    1320

gccatccagc acgagagcgg catcctcggc cctctcctgt acggagaggt cggagacaca    1380

ctcctcatca tttttaagaa ccaggcctcc cggccctaca acatctatcc tcatggcatc    1440

accgatgtga ggcctctgta cagcaggagg ctgcccaaag gagtcaagca cctgaaagat    1500

ttccctatcc tccccggaga gatcttcaag tacaaatgga ccgtcaccgt ggaggacggc    1560

cccacaaagt ccgatccccg gtgcctgacc cggtactatt ccagctttgt caacatggag    1620

cgggacctcg ccagcggcct gatcggccct ctgctcatct gctataagga gtccgtcgat    1680

cagaggggaa accagatcat gtccgataag cggaatgtga tcctgttttc cgtgtttgac    1740

gaaaaccggt cctggtatct gaccgagaac atccagcggt ttctgcctaa ccccgctggc    1800

gtgcaactgg aagatcctga gttccaggct tccaatatca tgcacagcat caacggctac 1860

gtcttcgaca gcctgcagct gtccgtctgc ctgcacgaag tcgcctactg gtacattctc 1920

agcattggcg cccagacaga cttcctgtcc gtctttttct ccggctatac cttcaaacat 1980

aagatggtct acgaggatac cctcacactg ttccctttca gcggcgaaac cgtgtttatg 2040

agcatggaaa atcccggcct gtggatttta ggatgccaca actccgattt caggaatagg 2100

ggcatgacag ctctcctcaa agtgtcctcc tgcgacaaga acacaggcga ctactacgag 2160

gacagctatg aggatatttc cgcctacctg ctcagcaaga ataatgccat cgagcctagg 2220

tccttctccc aaaactcccg gcatcccagc acccggcaga agcagttcaa tgccaccaca 2280

attcctgaaa atagaacaac cctgcagtcc gaccaggagg aaatcgacta cgatgacacc 2340

atctccgtcg agatgaagaa ggaagacttc gacatctatg acgaggacga aaaccagagc 2400

cctcggtcct ttcagaaaaa aacacggcat tactttatcg ctgccgtcga acggctgtgg 2460

gactacggca tgagctcctc cccccatgtg ctccggaatc gggctcaatc cggctccgtg 2520

cctcagttta agaaggtggt gtttcaagag ttcaccgacg gcagctttac acagcccctg 2580

tacaggggag agctgaacga gcacctggga ctgctcggac cttacattcg ggccgaagtc 2640

gaggataaca tcatggtgac ctttaggaat caggcttccc ggccttactc cttctactcc 2700

agcctcatct cctacgaaga agaccaacgg cagggcgccg agcctcggaa gaatttcgtg 2760

aagcccaatg aaaccaaaac atacttttgg aaagtccagc accacatggc ccctaccaag 2820

gatgagttcg actgcaaagc ctgggcctat ttctccgatg tggatctgga gaaggacgtc 2880

cattccggac tgatcggccc cctcctcgtc tgccacacca acaccctcaa tcctgcccac 2940

ggcaggcagg tgacagtcca ggagttcgcc ctgtttttca ccatctttga cgagaccaag 3000

tcctggtact ttaccgagaa tatggaaagg aattgcaggg ccccctgcaa cattcagatg 3060

gaggacccca ccttcaaaga gaactaccgg tttcatgcca ttaatggata catcatggac 3120

acactgcccg gcctggtgat ggctcaggac caacggattc ggtggtacct gctgagcatg 3180

ggctccaacg aaaatatcca cagcatccac tttagcggcc acgtgtttac cgtgcggaag 3240

aaggaggagt ataaaatggc cctctacaac ctctatcccg gcgtgttcga aacagtcgag 3300

atgctcccta gcaaggccgg aatctggagg gtggaatgcc tgattggcga acatctgcac 3360

gccggaatga gcaccctctt cctggtctac agcaacaaat gtcagacccc tctgggcatg 3420

gcttccggcc acatcaggga ctttcagatt accgctagcg gacaatatgg ccagtgggcc 3480

cctaaactcg ctaggctgca ctacagcgga agcatcaacg cctggtccac aaaggaacct 3540

ttctcctgga tcaaggtgga tctcctcgcc cctatgatca tccatggcat caagacccaa 3600

ggcgctaggc agaagtttag ctccctgtac atcagccagt ttatcatcat gtactccctc 3660

gacggcaaga agtggcagac ataccgggga aactccacag gcaccctgat ggtgttcttt 3720

ggcaacgtgg acagcagcgg aattaagcat aatattttca accctcctat catcgccagg 3780

tacatccggc tgcaccctac acactacagc atcaggagca ccctgaggat ggagctgatg 3840

ggctgcgatc tgaatagctg ctccatgccc ctcggcatgg agagcaaggc tatttccgac 3900

gcccaaatta ccgccagctc ctatttcacc aacatgttcg ccacatggag ccctagcaaa 3960

gctcggctcc atctccaggg caggtccaac gcctggcggc ctcaggtgaa taatcccaaa 4020

gaatggctgc aggtcgactt ccagaagaca atgaaagtga ccggcgtgac cacccaggga 4080

gtcaaaagcc tcctcaccag catgtacgtc aaagagttcc tgatcagctc ctcccaggac 4140

ggacatcaat ggacactctt ttttcagaac ggcaaggtga aggtgttcca gggcaaccag 4200

gattccttta cccctgtggt caactcctta gatcctcccc tcctgacacg gtacctgcgg 4260

attcaccctc agtcctgggt gcatcagatc gctctgagga tggaagtgct cggctgcgaa 4320

gcccaagacc tgtacggatc cggtggcggt ggctccggtg gaggcggaag cggcggtgga 4380

ggatcaggcg gtggaggtag cggcggaggc ggtagctcca gctctagtaa agctccccct 4440

ccttccctgc cctcaccctc aagactgcct ggaccttccg acactcccat cctgccacag 4500

gtggagtgcc ctccatgtcc agcaccccct gtcgcaggtc catctgtgtt cctgtttcca 4560

cccaagccta aagaccagct gatgatctcc cgcaccccag aagtcacctg tgtggtcgtg 4620

gatgtgagcc atgaagaccc cgaggtccag ttcaattggt acgtggatgg cgtcgaggtg 4680

cacaacgcta agacaaaacc tagagaagag cagttcaact ctacctttcg cgtcgtgagt 4740

gtgctgacag tcgtgcacca ggactggctg aatggcaagg agtataagtg caaagtgagc 4800

aacaaaggac tgcctgcctc aatcgaaaag actatttcca agaccaaagg acagccaaga 4860

gagccccagg tgtacaccct gcctccaagc cgcgaagaga tgactaaaaa tcaggtctct 4920

ctgacctgtc tggtgaaggg gttttatcct agtgatatcg ccgtggaatg ggagtcaaac 4980

ggtcagccag agaacaatta caagaccaca ccccctatgc tggacagcga tgggtctttc 5040

tttctgtata gcaaactgac agtggacaag tctcggtggc agcagggtaa cgtcttctct 5100

tgcagtgtgc tgcacgaagc actgcacaat cattacaccc agaagtcact gtcactgagc 5160

ccaggaaaa 5169

<210> 11
<211> 1716
<212> PRT
<213> Artificial sequence

<220>
<223> amino acid sequence of DS-F8 fusion protein

<400> 11

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Pro Pro Val Leu Lys Arg His
            740                 745                 750

Gln Arg Glu Ile Thr Arg Thr Thr Leu Gln Ser Asp Gln Glu Glu Ile

        755                 760                 765

Asp Tyr Asp Asp Thr Ile Ser Val Glu Met Lys Lys Glu Asp Phe Asp
    770                 775                 780

Ile Tyr Asp Glu Asp Glu Asn Gln Ser Pro Arg Ser Phe Gln Lys Lys
785                 790                 795                 800

Thr Arg His Tyr Phe Ile Ala Ala Val Glu Arg Leu Trp Asp Tyr Gly
                805                 810                 815

Met Ser Ser Ser Pro His Val Leu Arg Asn Arg Ala Gln Ser Gly Ser
            820                 825                 830

Val Pro Gln Phe Lys Lys Val Val Phe Gln Glu Phe Thr Asp Gly Ser
        835                 840                 845

Phe Thr Gln Pro Leu Tyr Arg Gly Glu Leu Asn Glu His Leu Gly Leu
    850                 855                 860

Leu Gly Pro Tyr Ile Arg Ala Glu Val Glu Asp Asn Ile Met Val Thr
865                 870                 875                 880

Phe Arg Asn Gln Ala Ser Arg Pro Tyr Ser Phe Tyr Ser Ser Leu Ile
                885                 890                 895

Ser Tyr Glu Glu Asp Gln Arg Gln Gly Ala Glu Pro Arg Lys Asn Phe
            900                 905                 910

Val Lys Pro Asn Glu Thr Lys Thr Tyr Phe Trp Lys Val Gln His His
        915                 920                 925

Met Ala Pro Thr Lys Asp Glu Phe Asp Cys Lys Ala Trp Ala Tyr Phe
    930                 935                 940

Ser Asp Val Asp Leu Glu Lys Asp Val His Ser Gly Leu Ile Gly Pro
945                 950                 955                 960

Leu Leu Val Cys His Thr Asn Thr Leu Asn Pro Ala His Gly Arg Gln
                965                 970                 975

Val Thr Val Gln Glu Phe Ala Leu Phe Phe Thr Ile Phe Asp Glu Thr
            980                 985                 990

Lys Ser Trp Tyr Phe Thr Glu Asn Met Glu Arg Asn Cys Arg Ala Pro
        995                 1000                1005

Cys Asn Ile Gln Met Glu Asp Pro Thr Phe Lys Glu Asn Tyr Arg
    1010                1015                1020

Phe His Ala Ile Asn Gly Tyr Ile Met Asp Thr Leu Pro Gly Leu
    1025                1030                1035

Val Met Ala Gln Asp Gln Arg Ile Arg Trp Tyr Leu Leu Ser Met
    1040                1045                1050

Gly Ser Asn Glu Asn Ile His Ser Ile His Phe Ser Gly His Val
    1055                1060                1065

Phe Thr Val Arg Lys Lys Glu Glu Tyr Lys Met Ala Leu Tyr Asn
    1070                1075                1080

Leu Tyr Pro Gly Val Phe Glu Thr Val Glu Met Leu Pro Ser Lys
    1085                1090                1095

Ala Gly Ile Trp Arg Val Glu Cys Leu Ile Gly Glu His Leu His
    1100                1105                1110

Ala Gly Met Ser Thr Leu Phe Leu Val Tyr Ser Asn Lys Cys Gln
    1115                1120                1125

Thr Pro Leu Gly Met Ala Ser Gly His Ile Arg Asp Phe Gln Ile
    1130                1135                1140

Thr Ala Ser Gly Gln Tyr Gly Gln Trp Ala Pro Lys Leu Ala Arg
    1145                1150                1155

Leu His Tyr Ser Gly Ser Ile Asn Ala Trp Ser Thr Lys Glu Pro
    1160                1165                1170

Phe Ser Trp Ile Lys Val Asp Leu Leu Ala Pro Met Ile Ile His
    1175                1180                1185

Gly Ile Lys Thr Gln Gly Ala Arg Gln Lys Phe Ser Ser Leu Tyr
    1190                1195                1200

Ile Ser Gln Phe Ile Ile Met Tyr Ser Leu Asp Gly Lys Lys Trp
    1205                1210                1215

Gln Thr Tyr Arg Gly Asn Ser Thr Gly Thr Leu Met Val Phe Phe
    1220                1225                1230

Gly Asn Val Asp Ser Ser Gly Ile Lys His Asn Ile Phe Asn Pro
    1235                1240                1245

Pro Ile Ile Ala Arg Tyr Ile Arg Leu His Pro Thr His Tyr Ser
    1250                1255                1260

Ile Arg Ser Thr Leu Arg Met Glu Leu Met Gly Cys Asp Leu Asn
    1265                1270                1275

Ser Cys Ser Met Pro Leu Gly Met Glu Ser Lys Ala Ile Ser Asp
    1280                1285                1290

Ala Gln Ile Thr Ala Ser Ser Tyr Phe Thr Asn Met Phe Ala Thr
    1295                1300                1305

Trp Ser Pro Ser Lys Ala Arg Leu His Leu Gln Gly Arg Ser Asn
    1310                1315                1320

Ala Trp Arg Pro Gln Val Asn Asn Pro Lys Glu Trp Leu Gln Val
    1325                1330                1335

Asp Phe Gln Lys Thr Met Lys Val Thr Gly Val Thr Thr Gln Gly
    1340                1345                1350

Val Lys Ser Leu Leu Thr Ser Met Tyr Val Lys Glu Phe Leu Ile
    1355                1360                1365

Ser Ser Ser Gln Asp Gly His Gln Trp Thr Leu Phe Phe Gln Asn
    1370                1375                1380

Gly Lys Val Lys Val Phe Gln Gly Asn Gln Asp Ser Phe Thr Pro
    1385                1390                1395

Val Val Asn Ser Leu Asp Pro Pro Leu Leu Thr Arg Tyr Leu Arg
    1400                1405                1410

Ile His Pro Gln Ser Trp Val His Gln Ile Ala Leu Arg Met Glu
    1415                1420                1425

Val Leu Gly Cys Glu Ala Gln Asp Leu Tyr Gly Ser Gly Gly Gly
    1430                1435                1440

Gly Ser Gly Gly Gly Gly Ser Gly Gly Gly Gly Ser Gly Gly Gly
    1445                1450                1455

Gly Ser Gly Gly Gly Gly Ser Ser Ser Ser Ser Lys Ala Pro Pro
    1460                1465                1470

Pro Ser Leu Pro Ser Pro Ser Arg Leu Pro Gly Pro Ser Asp Thr
    1475                1480                1485

Pro Ile Leu Pro Gln Val Glu Cys Pro Pro Cys Pro Ala Pro Pro
    1490                1495                1500

Val Ala Gly Pro Ser Val Phe Leu Phe Pro Pro Lys Pro Lys Asp
    1505                1510                1515

Gln Leu Met Ile Ser Arg Thr Pro Glu Val Thr Cys Val Val Val
    1520                1525                1530

Asp Val Ser His Glu Asp Pro Glu Val Gln Phe Asn Trp Tyr Val
    1535                1540                1545

Asp Gly Val Glu Val His Asn Ala Lys Thr Lys Pro Arg Glu Glu
    1550                1555                1560

Gln Phe Asn Ser Thr Phe Arg Val Val Ser Val Leu Thr Val Val
    1565                1570                1575

His Gln Asp Trp Leu Asn Gly Lys Glu Tyr Lys Cys Lys Val Ser
    1580                1585                1590

Asn Lys Gly Leu Pro Ala Ser Ile Glu Lys Thr Ile Ser Lys Thr
    1595                1600                1605

Lys Gly Gln Pro Arg Glu Pro Gln Val Tyr Thr Leu Pro Pro Ser
    1610                1615                1620

Arg Glu Glu Met Thr Lys Asn Gln Val Ser Leu Thr Cys Leu Val
    1625                1630                1635

Lys Gly Phe Tyr Pro Ser Asp Ile Ala Val Glu Trp Glu Ser Asn
    1640                1645                1650

Gly Gln Pro Glu Asn Asn Tyr Lys Thr Thr Pro Pro Met Leu Asp
    1655                1660                1665

Ser Asp Gly Ser Phe Phe Leu Tyr Ser Lys Leu Thr Val Asp Lys
    1670                1675                1680

Ser Arg Trp Gln Gln Gly Asn Val Phe Ser Cys Ser Val Leu His
    1685                1690                1695

Glu Ala Leu His Asn His Tyr Thr Gln Lys Ser Leu Ser Leu Ser
    1700                1705                1710

Pro Gly Lys

    1715

<210>  12
<211>  16
<212>  PRT
<213>  Artificial sequence

<220>
<223>  flexible peptide linker

<400>  12

Gly Ser Gly Gly Gly Ser Gly Gly Gly Gly Ser Gly Gly Gly Gly Ser
1               5                   10                  15

<210>  13
<211>  27
<212>  PRT
<213>  Artificial sequence

<220>
<223>  flexible peptide linker

<400>  13

Gly Ser Gly Gly Gly Gly Ser Gly Gly Gly Gly Ser Gly Gly Gly Gly
1               5                   10                  15

Ser Gly Gly Gly Gly Ser Gly Gly Gly Gly Ser
            20                  25

<210>  14
<211>  20
<212>  PRT
<213>  Artificial sequence

<220>
<223>  flexible peptide linker

<400>  14

Gly Gly Gly Gly Ser Gly Gly Gly Gly Ser Gly Gly Gly Gly Ser Gly
1               5                   10                  15

Gly Gly Gly Ser
            20

<210>  15
<211>  37
<212>  PRT
<213>  Artificial sequence

<220>
<223>  flexible peptide linker

<400>  15

Gly Ser Gly Gly Gly Gly Ser Gly Gly Gly Gly Ser Gly Gly Gly Gly
1               5                   10                  15

Ser Gly Gly Gly Gly Ser Gly Gly Gly Gly Ser Gly Gly Gly Gly Ser
            20                  25                  30

Gly Gly Gly Gly Ser
        35

<210>  16
<211>  20
<212>  PRT
<213>  Artificial sequence

<220>
<223>  flexible peptide linker

<400>  16

Gly Gly Gly Ser Gly Gly Gly Ser Gly Gly Gly Ser Gly Gly Gly Ser
1               5                   10                  15

Gly Gly Gly Ser
            20

<210>  17
<211>  33
<212>  PRT
<213>  Homo sapiens

<400>  17

Pro Arg Phe Gln Asp Ser Ser Ser Ser Lys Ala Pro Pro Pro Ser Leu
1               5                   10                  15

Pro Ser Pro Ser Arg Leu Pro Gly Pro Ser Asp Thr Pro Ile Leu Pro
            20                  25                  30

Gln

<210>  18
<211>  28
<212>  PRT
<213>  Homo sapiens

<400>  18

Ser Ser Ser Ser Lys Ala Pro Pro Pro Ser Leu Pro Ser Pro Ser Arg
1               5                   10                  15

Leu Pro Gly Pro Ser Asp Thr Pro Ile Leu Pro Gln
            20                  25

<210>  19
<211>  10
<212>  PRT
<213>  Homo sapiens

<400>  19

Ser Ser Ser Ser Lys Ala Pro Pro Pro Ser
1               5                   10


<210>  20
<211>  14
<212>  PRT
<213>  Homo sapiens

<400>  20

Ser Arg Leu Pro Gly Pro Ser Asp Thr Pro Ile Leu Pro Gln
1               5                   10